# EUROPEAN PATENT APPLICATION

(11) **EP 4 134 084 A2**
(43) Date of publication of application: **15.02.2023**
(21) Application number: 22189732.5
(22) Date of filing: 10.08.2022
(51) Int. Cl.: A61K 31/7105, C12N 15/85, C12N 5/10, A61K 48/00

(54) **TISSUE-SPECIFICALLY EXPRESSED CIRCULAR RNA MOLECULE AND APPLICATION THEREOF**

(30) Priority: 13.08.2021 CN 202110931829
(71) Applicant: Suzhou Curemed Biomedical Technology Co. Ltd, Suzhou, Jiangsu 215123 (CN)
(72) Inventor: ZUO, Chijian, Suzhou, 215123 (CN); QIU, Zonghao, Suzhou, 215123 (CN); YANG, Jiali, Suzhou, 215123 (CN); ZHU, Jiafeng, Suzhou, 215123 (CN); LI, Na, Suzhou, 215123 (CN); SUN, Zhenhua, Suzhou, 215123 (CN)
(74) Representative: Heubeck, Christian

(57) **Abstract**

The present disclosure belongs to the technical field of bio-medicine, particularly, the present disclosure relates to a tissue-specifically expressed circular RNA molecule, a cyclization precursor RNA molecule, a recombinant nucleic acid molecule, a recombinant expression vector, a recombinant host cell, a composition, a pharmaceutical preparation and application thereof in preparation of a drug for preventing or treating diseases, as well as a method for preventing or treating diseases. The circular RNA molecule provided by the present disclosure operably links a coding region to an expression regulatory element, realizing specific high expression of a target polypeptide in target cells or target tissues, and low expression of the target polypeptide in non-target cells or non-target tissues, and having high tissue expression specificity, thereby providing a safe and effective treatment strategy for clinical targeted therapy of diseases such as tumors.

## Description

### CROSS-REFERENCE TO RELATED APPLICATIONS

The present application claims priority to the Chinese Patent Application No. 202110931829.3 filled on August 13, 2021, which is incorporated herein by reference in its entirety.

### TECHNICAL FIELD

The present disclosure belongs to the technical field of bio-medicine, particularly, the present disclosure relates to a tissue-specifically expressed circular RNA molecule, a cyclization precursor RNA molecule, a recombinant nucleic acid molecule, a recombinant expression vector, a recombinant host cell, a composition, a pharmaceutical preparation and application thereof in preparation of a drug for preventing or treating diseases, as well as a method for preventing or treating diseases.

### BACKGROUND

With the emergence of synthetic mRNA as an emerging tool for in-vivo protein expression, mRNA therapy brings about more possibilities for diagnosis and treatment of tumors, genetic defect disease and epidemic disease, etc. Because mRNA can be synthesized in-vitro, compared with other current biotechnological treatment means, synthesis of the mRNA is more efficient, fast, open and low in cost. But there are still many technical difficulties in medication formation of the mRNA, such as stability of mRNA, translation efficiency, immunogenicity and delivery. With advances in technology, these technical obstacles are slowly fading away, for example, capping at a 5' end of linear mRNA, and adding a tail of polyA at a 3' end can significantly increase in-vivo half-life of the mRNA, the translation efficiency can be increased by codon optimization, and immunogenicity of the mRNA can be decreased by modification to a specific nucleotide. Researches have reported that when circular RNA contains an internal ribosome entry site (IRES), the circular RNA can bind to small ribosomal subunits to initiate ribosomal translation, namely the circular RNA can encode protein synthesis. Such circular RNA which can encode protein synthesis is known as circular mRNA. Due to the end-to-end characteristic of the circular mRNA, RNaseR can not effectively excise the circular mRNA. Thus, compared with the linear mRNA, the circular mRNA has a very long half-life namely more stable expression. Researches have shown that mRNA encoding a same protein can be stably expressed for 48 hours or above in a circular form, and can only be expressed for less than 20 hours in a linear form. Therefore, the circular mRNA, as a novel mRNA therapy tool, has a great development prospect in the field of gene therapy. Especially in the related fields such as vaccines, tumor immunotherapy and protein supplement, the circular mRNA shows a huge advantage.

The circular RNA can be formed by linking the linear RNA molecule by a T4 ligase, a chemical method or a ribozyme method to form a circular form. Wherein, T4 DNA ligase (T4 Dnl), T4 RNA ligasel (T4 Rnl1) and T4 RNA ligase II (T4 Rnl2) catalyze the formation of a new phosphodiester bond at a 5' end and a 3' end of a linear RNA precursor to link a head end with a tail end of the linear RNA. The T4 ligase method is an earliest RNA cyclization method, but it is also easy to form a polymer byproduct. The chemical method is to form the circular RNA by modifying two ends of the linear RNA in connection with a condensation reaction, but the chemical method needs to introduce certain environmentally hazardous and explosive chemicals, such as cyanogen bromide, 1-ethyl-3-(3-dimethylaminopropyl)carbodiimide hydrochloride, etc., hence there is a large limitation in application of therapeutic circular mRNA. The ribozyme method is to make use of some endonucleases existing in nature which can specifically recognize and specifically cleave small molecule RNA, the endonuclease itself is also RNA and able to cleave a phosphodiester bond of a substrate RNA in a reversible way. Due to such characteristic, the ribozyme can also catalyze cyclization of linear RNA. The ribozyme method is more effective to cyclize some long linear RNAs, but such cyclization method is very unstable, thus it also needs further improvement. In addition, a natural class I intron system can also form circular intron RNA by cleavage and ligation reactions, which is known as a group I permuted intron-exon self-cleavage system (a PIE system), and such PIE system has been successfully used in constructing circular RNA expressed by eukaryotic protein.

At present, the circular RNA molecule shows an important application value in clinical disease treatment, however, due to limitation by the mode of administration, after being delivered into the body, the circular mRNA also exhibits a high abundance expression (e.g., usually having a high abundance trend in liver) in a non-diseased part, which causes certain damage to normal tissues and organs of the body. Therefore, how to realize a tissue specific expression regulation for the circular RNA molecule is an important issue that needs to be urgently solved in the field.

### SUMMARY

### Problems to be solved bv the invention

In view of the problems existing in the prior art, for example, a circular RNA molecule cannot realize differential expression among different tissues, after the circular RNA molecule is delivered to the body, it is easy to generate high abundance expression in a non-diseased tissue, causing injury to healthy tissues and organs. For this reason, the present disclosure provides a tissue-specifically expressed circular RNA molecule, which operably links a coding region to an expression regulatory element, realizing specific high expression of a target polypeptide in target cells or target tissues, and low expression of the target polypeptide in non-target cells or non-target tissues, and further realizing tissue specific expression of the circular RNA molecule, thereby providing a safe and effective treatment strategy for clinical targeted treatment of diseases such as tumor.

### Solution for solving the problems

(1) A circular RNA molecule, wherein the circular RNA molecule includes a coding region which encodes a target polypeptide, and an expression regulatory element which is operably linked to the coding region; the expression regulatory element has the characteristics shown in any one in the group consisting of (i)-(ii):
   (i) enhancing expression of the target polypeptide in target cells or target tissues, and
   (ii) attenuating expression of the target polypeptide in non-target cells or non-target tissues.
(2) The circular RNA molecule according to (1), wherein the circular RNA molecule includes an expression regulatory element as shown in (ii); wherein the expression regulatory element includes one or more miRNA recognition regions which are hybridized with a sequence of miRNA; preferably, an expression level of the miRNA in the non-target cells or non-target tissues is higher than an expression level of the miRNA in the target cells or target tissues; more preferably, an expression level of the miRNA in non-tumor cells or non-tumor tissues is higher than an expression level of the miRNA in tumor cells or tumor tissues.
(3) The circular RNA molecule according to (1) or (2), wherein the expression regulatory element includes one or more miRNA recognition regions which are hybridized with sequences of one or more miRNAs as follows:
   miR-122, miR-152, miR-148, miR-194, miR-199, miR-215, miR-18, miR-20, miR-24, miR-30b, miR-30c, miR-32, miR-141, miR-192, miR-193, miR-194, miR-200b, miR-204, miR-18, miR-19a, miR-20, miR-24, miR-32, miR-126, miR-133, miR-141, miR-193, miR-200b, miR-213, miR-1, miR-133, miR-143, miR-149, miR-206, miR-208, miR-7, miR-9, miR-124, miR-125, miR-128, miR-132, miR-135, miR-137, miR-139, miR-149, miR-153, miR-183, miR-190, miR-219, miR-16, miR-21, miR-24, miR-27, miR-99a, miR-127, miR-132, miR-142, miR-151, miR-181, miR-189, miR-212, miR-223, miR-17, and miR-92;
   preferably, the miRNA recognition region includes a sequence shown in any one of (a₁)-(a₂):
      (a₁) is a nucleotide sequence shown in any one of SEQ ID NOs: 2-66;
      (a₂) is a sequence having at least 90%, optionally at least 95%, preferably at least 97%,
   more preferably at least 98%, most preferably at least 99% sequence identity with the nucleotide sequence shown in (a₁);
   optionally, the expression regulatory element comprises one or more miRNA recognition regions shown in any one of (a₁) or (a₂); the expression regulatory element is selected from a sequence in which one or more miRNA recognition regions shown in any one of (a₁) or (a₂) are inserted into a nucleotide sequence shown in SEQ ID NO: 1; preferably, a nucleotide sequence of the expression regulatory element is a sequence in which 1, 2 or 3 miRNA recognition regions shown in any one of (a₁) or (a₂) are inserted into the nucleotide sequence shown in in SEQ ID NO: 1; more preferably, the miRNA recognition region is inserted between 88th and 89th nucleotides, between 48th and 49th nucleotides, or between 9th and 10th nucleotides shown in SEQ ID NO: 1.
(4) The circular RNA molecule according to any one of (1)-(3), wherein the circular RNA molecule includes a translation initiation element which is operably linked to the coding region, and an expression regulatory element shown in (ii); optionally, the translation initiation element is located at the upstream of the coding region, and the expression regulatory element shown in (ii) is located at the downstream of the encoding region;
   preferably, the translation initiation element is an IRES element;
   preferably, the IRES element includes any one of the following group of (iii)-(vi):
      (iii) a nucleotide sequence including one or more sequences selected from SEQ ID NOs: 78-81 in any combination;
      (iv) a nucleotide sequence including a reverse complementary sequence of any sequence shown in SEQ ID NOs: 78-81;
      (v) a reverse complementary sequence of a sequence which is capable of being hybridized with the nucleotide sequence shown in (i) or (ii) under highly stringent hybridization conditions or extremely highly stringent hybridization conditions; and
      (vi) a sequence having at least 90%, optionally at least 95%, preferably at least 97%, more preferably at least 98%, most preferably at least 99% sequence identity with the nucleotide sequence shown in (iii) or (iv);
   preferably, the IRES element is selected from any one of (b₁)-(b₇):
      (b₁) a nucleotide sequence including a sequence shown in SEQ ID NO: 78;
      (b₂) a nucleotide sequence including a sequence shown in SEQ ID NO: 79;
      (b₃) a nucleotide sequence including a sequence shown in SEQ ID NO: 80;
      (b₄) a nucleotide sequence including a sequence shown in SEQ ID NO: 81;
      (b₅) a nucleotide sequence including a sequence shown in SEQ ID NO: 82;
      (b₆) a nucleotide sequence including a sequence shown in SEQ ID NO: 83; and
      (b₇) a nucleotide sequence including a sequence shown in SEQ ID NO: 84.
(5) The circular RNA molecule according to (4), wherein the circular RNA molecule further includes one or more elements as follows: a 5' spacer region, a 3' spacer region, a second exon, a first exon, and an expression regulatory element shown in (i).
(6) The circular RNA molecule according to (5), wherein the circular RNA molecule includes a 5' spacer region located at the upstream of the translation initiation element, and a 3' spacer region located at the downstream of the expression regulatory element;
   preferably, the 5' spacer region includes a sequence shown in any one of (c₁)-(c₂):
      (c₁) a nucleotide sequence shown in any one of SEQ ID NOs: 85-86;
      (c₂) a sequence having at least 90%, optionally at least 95%, preferably at least 97%, more preferably at least 98%, most preferably at least 99% sequence identity with the nucleotide sequence shown in (c₁);
   preferably, the 3' spacer region includes a sequence shown in any one of (d₁)-(d₂):
      (d₁) a nucleotide sequence shown in any one of SEQ ID NOs: 87-89;
      (d₂) a sequence having at least 90%, optionally at least 95%, preferably at least 97%, more preferably at least 98%, most preferably at least 99% sequence identity with the nucleotide sequence shown in (d₁).
(7) The circular RNA molecule according to (5), wherein the circular RNA molecule further includes a second exon located at the upstream of the 5' spacer region, and a first exon located at the downstream of the 3' spacer region;
   preferably, the second exon includes a sequence shown in any one of (e₁)-(e₂):
      (e₁) a nucleotide sequence shown in SEQ ID NO: 91;
      (e₂) a sequence having at least 90%, optionally at least 95%, optionally at least 97%, more optionally at least 98%, most optionally at least 99% sequence identity with the nucleotide sequence shown in (e₁);
   preferably, the first exon includes a sequence shown in any one of (f₁)-(f₂):
      (f₁) a nucleotide sequence as shown in SEQ ID NO: 90;
      (f₂) a sequence having at least 90%, optionally at least 95%, optionally at least 97%, more optionally at least 98%, most optionally at least 99% sequence identity with the nucleotide sequence shown in (f₁).
(8) The circular RNA molecule according to any one of (1)-(7), wherein the target polypeptide is a polypeptide having an activity of disease prevention or treatment, preferably a polypeptide having an activity of tumor prevention or treatment; optionally, the polypeptide having the activity of tumor prevention or treatment is one or more selected from: cytokine, an antigen-binding fragment, and an immune checkpoint inhibitor;
   optionally, the cytokine is selected from one or more of IL, IFN, TNF, GM-CSF, and M-CSF;
   optionally, the antigen-binding fragment specifically binds to one or more tumor antigens as follows: CD19, CD20, CD22, CD30, CD33, CD38, CD123, CD138, CD171, AFP, CEA, PSCA, GD2, NKG2D, BCMA, EGFR, Her2, EGFRvIII, CD171, FAP, IL13Rα2, VEGFR1, VEGFR2, GPC-3, Mesothelin, claudin 18.2, EpCAM, MUC1, MUC16, EPHA2, EPHA3, CD133, and PSMA;
   optionally, the immune checkpoint inhibitor is one or more immune checkpoint protein inhibitors as follows: PD-1, PD-L1, PDL2, CTLA4, LAG3, TIM3, TIGIT and CD103.
(9) The circular RNA molecule according to any one of (1)-(7), wherein the circular RNA molecule includes a sequence shown in any one of (g₁)-(g₂):
   (g₁) nucleotide sequences shown in SEQ ID NO: 73, and SEQ ID NOs: 76-77;
   (g₂) a sequence having at least 90%, optionally at least 95%, optionally at least 97%, more optionally at least 98%, most optionally at least 99% sequence identity with the nucleotide sequences shown in (g₁).
(10) A cyclization precursor RNA molecule, wherein the cyclization precursor RNA molecule is cyclized to form the circular RNA molecule according to any one of (1)-(9);
   optionally, the cyclization precursor RNA molecule includes one or more elements as follows:
   a 5' homologous arm, a 3' intron, a second exon, a 5' spacer region, a translation initiation element, an expression regulatory element, a coding region, a 3' spacer region, a first exon, a 5' intron and a 3' homologous arm.
(11) A recombinant nucleic acid molecule, wherein the recombinant nucleic acid molecule is transcribed to form the cyclization precursor RNA molecule according to (10).
(12) A recombinant expression vector, wherein the recombinant expression vector includes the recombinant nucleic acid molecule according to (11).
(13) A recombinant host cell, wherein the recombinant host cell includes the circular RNA molecule according to any one of (1)-(9), the cyclization precursor RNA molecule according to (10), the recombinant nucleic acid molecule according to (11), or the recombinant expression vector according to (12).
(14) A pharmaceutical preparation or composition, wherein the pharmaceutical preparation or composition includes the circular RNA molecule according to any one of (1)-(9), the cyclization precursor RNA molecule according to (10), the recombinant nucleic acid molecule according to (11), the recombinant expression vector according to (12), or the recombinant host cell according to (13); optionally, the pharmaceutical preparation or composition further includes one or more pharmaceutically acceptable carriers; preferably, the pharmaceutically acceptable carrier is a liposome material which encapsulates the circular RNA molecule, the cyclization precursor RNA molecule, the recombinant nucleic acid molecule, the recombinant expression vector, or the recombinant host cell.
(15) The pharmaceutical preparation or composition according to (14), wherein the pharmaceutical preparation is a tablet, capsule, injection, spray, granule, powder, suppository, pill, cream, paste, gel, powder, oral solution, inhalant, suspension or dry suspension;
   preferably, the pharmaceutical preparation is the injection;
   preferably, the pharmaceutical preparation is a pharmaceutical preparation which is intratumorally delivered to tumors.
(16) Application of the circular RNA molecule according to any one of (1)-(9), the cyclization precursor RNA molecule according to (10), the recombinant nucleic acid molecule according to (11), the recombinant expression vector according to (12), the recombinant host cell according to (13), or the composition or pharmaceutical preparation according to any one of (14)-(15) in preparation of a drug for preventing or treating diseases;
   preferably application in preparation of a drug for preventing or treating tumors, more preferably application in preparation of an intratumoral injection for preventing or treating tumors.
(17) A method for preventing or treating diseases, including administering the circular RNA molecule according to any one of (1)-(9), the cyclization precursor RNA molecule according to (10), the recombinant nucleic acid molecule according to (11), the recombinant expression vector according to (12), the recombinant host cell according to (13) or the composition or pharmaceutical preparation according to any one of (14)-(15) to a subject;
   optionally, the administration is selected from oral administration, intraperitoneal administration, intravenous administration, intra-arterial administration, intramuscular administration, intradermal administration, subcutaneous administration, transdermal administration, nasal administration, transrectal administration, intratumoral injection, intraluminal retention of tumor, nerve intrathecal injection, intrathecal injection or systemic administration; preferably intratumoral injection.

### Effects of the invention

In some embodiments, the circular RNA molecule provided by the present disclosure realizes specific high expression of the target polypeptide in target cells or target tissues, and low expression of the target polypeptide in non-target cells or non-target tissues by operably linking the coding region to the expression regulatory element, further realizes tissue specific expression of the circular RNA molecule, and effectively increases targeted expression and therapeutic effects of the circular RNA molecule, having a wide application prospect in clinical disease treatment.

In some embodiments, the circular RNA molecule provided by the present disclosure realizes tissue specific expression of the circular RNA molecule by introducing one or more miRNA recognition regions. The present disclosure first confirms that after introduction of the miRNA recognition regions, expression of the circular mRNA can be effectively inhibited without influencing the efficiency of a cyclization reaction of the circular RNA molecule. The circular RNA molecule of the present disclosure can realize high expression in the target cells or target tissues (e.g., tumor cells or tumor tissues), whereas the expression in non-target cells or non-target tissues (e.g. healthy tissues or healthy tissue cells) is inhibited, so as to reduce adverse reactions and side effects which possibly result from expression of the circular RNA molecule in healthy tissues.

In some embodiments, the circular RNA molecule provided by the present disclosure is encapsulated in a liposome material and delivered into the body, and is able to be specifically expressed in a specific tissue and organ, and there is no expression or very low expression in other tissues, so as to effectively solves the problem of strong hepatotoxicity due to high abundance expression in liver cells when the mRNA molecule is delivered by LNP, providing a safe and effective treatment strategy for clinical targeted treatment of diseases such as tumor with.

In some embodiments, the circular RNA molecule provided by the present disclosure is encapsulated in a liposome material and delivered into a tumor body, the circular RNA molecule can targetedly express a target polypeptide with therapeutic effects in tumor tissues, and there is low expression or no expression in other normal tissues and organs such as liver, etc., so as to achieve tumor targeting. Further, the circular RNA molecule is encapsulated in the liposome material, and is suitable to be used as an intratumor injection, enables local high expression of therapeutic polypeptides such as cytokine, an antigen-binding fragment, and an immune checkpoint inhibitor, and low expression or no expression in healthy cells and tissues such as liver cells and tissues, having high tumor targeting and low toxic and side effects, and thus the circular RNA molecule is a tumor immunotherapy molecule having a great application potential.

### BRIEF DESCRIPTION OF THE DRAWINGS

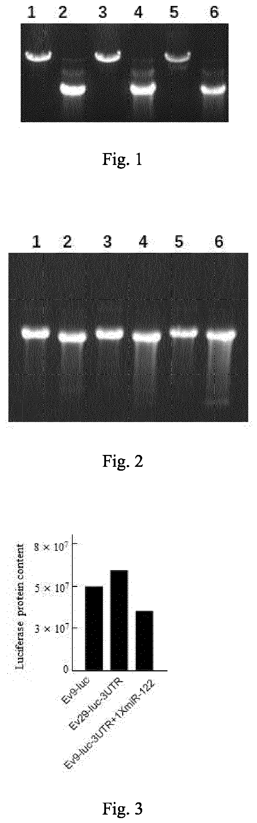

Fig. 1 shows plasmid template DNA, and agarose gel electrophoresis detection results of cyclization precursor RNA transcribed from the plasmid template DNA; in the figure, 1: pUC-EV29-LUC linearized plasmid template DNA, 2: pUC-EV29-LUC cyclization precursor RNA, 3: pUC-EV29-LUC-3'UTR linearized plasmid template DNA, 4: linear pUC-EV29-LUC-3'UTR precursor, 5: pUC-EV29-LUC-3'UTR+1×miR-122 linearized plasmid template DNA, and 6: linear pUC-EV29-LUC-3'UTR+1×miR-122 precursor.
Fig. 2 shows cyclization precursor RNA, and agarose gel electrophoresis detection results of circular RNA obtained by cyclization of the cyclization precursor RNA; in the figure, 1: EV29-LUC cyclization precursor RNA, 2: EV29-LUC circular RNA, 3: linear EV29-LUC-3'UTR precursor, 4: circular EV29-LUC-3'UTR, 5: linear EV29-LUC-3'UTR+1×miR-122 precursor, and 6: circular EV29-LUC-3'UTR+1×miR-122.
Fig. 3 shows detection results of the content of Luciferase protein expressed by circular EV29-LUC, circular EV29-LUC-3'UTR, and circular EV29-LUC-3'UTR+1×miR-122 in cells.

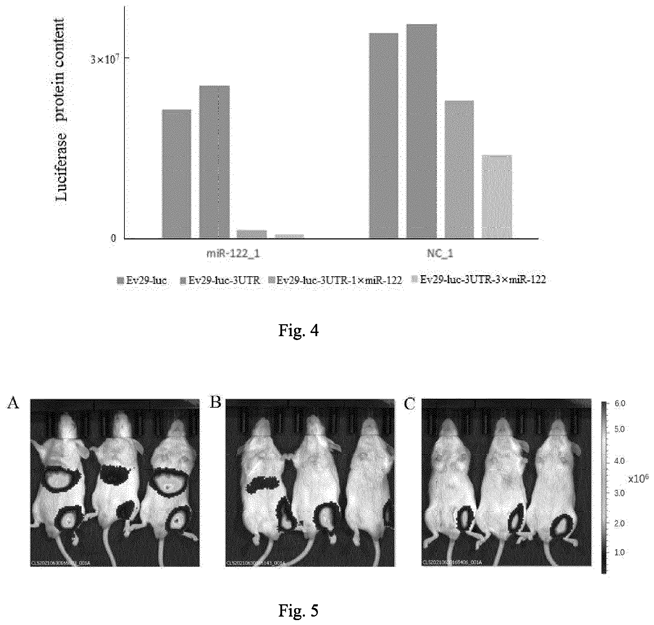

Fig. 4 shows detection results of the content of Luciferase protein expressed by circular EV29-LUC, circular EV29-LUC-3'UTR, circular EV29-LUC-3'UTR+1×miR-122, and circular EV29-LUC-3'UTR+3×miR-122 in cells.
Fig. 5 shows detection results of Luciferase protein expression mediated by a circular RNA molecule in specific sites of mice, wherein A-C successively represents Luciferase protein expression mediated by EV29-LUC-3'UTR, EV29-LUC-3'UTR+1xmiR-122, and EV29-LUC-3'UTR+3×miR-122.

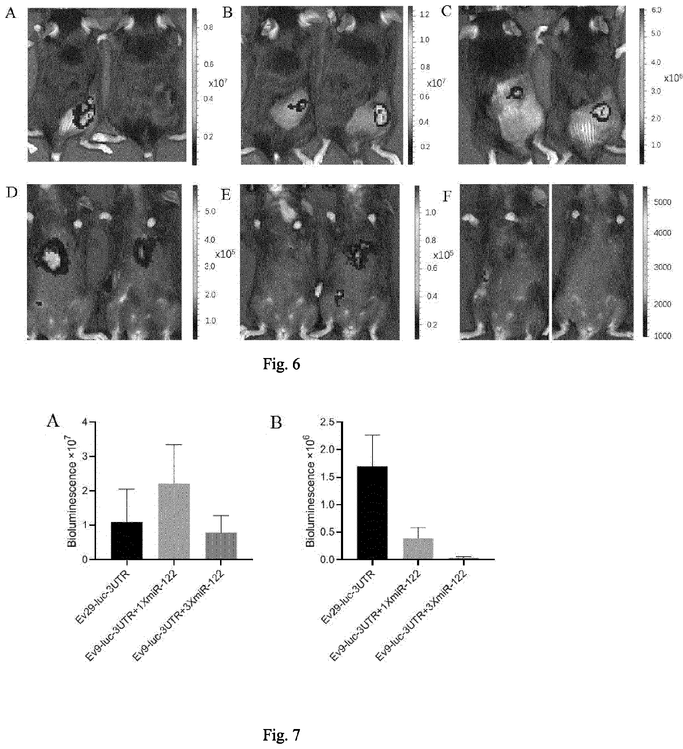

Fig. 6 shows detection results of Luciferase protein expression mediated by a circular RNA molecule in mouse tumor in situ and liver, wherein A-C successively represent detection results of Luciferase protein expression mediated by circular EV29-LUC-3'UTR, linear EV29-LUC-3'UTR+1xmiR-122 precursor, and EV29-LUC-3'UTR+3×miR-122 in mouse tumor in situ, and D-F successively represent detection results of Luciferase protein expression mediated by circular EV29-LUC-3'UTR, linear EV29-LUC-3'UTR+1×miR-122 precursor, and circular EV29-LUC-3'UTR+3×miR-122 in mouse liver.
Fig. 7 shows quantitative detection results of Luciferase protein expression mediated by a circular RNA molecule in mouse tumor in situ and liver. Wherein Fig. A represents quantitative detection results of Luciferase protein expression mediated by circular EV29-LUC-3'UTR, linear EV29-LUC-3'UTR+1×miR-122 precursor, and circular EV29-LUC-3'UTR+3×miR-122 in mouse tumor in situ, and Fig. B represents quantitative detection results of Luciferase protein expression mediated by circular EV29-LUC-3'UTR, linear EV29-LUC-3'UTR+1×miR-122 precursor, and circular EV29-LUC-3'UTR+3×miR-122 in mouse liver.

### DETAILED DESCRIPTION

### Definition

When used in combination with the term "include" in the claims and/or description, the word "a" or "an" can refer to "one", but can also refer to "one or more", "at least one" and "one or more than one".

As used in the claims and the description, the word "include", "have", "comprise" or "contain" is meant to be inclusive or open-ended, and does not exclude additional, unquoted elements or method steps.

Throughout the application document, the term "about" means that one value includes a standard deviation of an error of a device or method used in measurement of this value.

Although the content disclosed supports the definition of the term "or" only as a substitute as well as "and/or", the term "or" in the claims refers to "and /or" unless it is explicitly stated that there is only the substitute or substitutes are mutually exclusive.

As used in the present disclosure, the terms "polypeptide", "peptide" and "protein" are used interchangeably herein and represent an amino acid polymer of any length. This polymer can be linear or branched, it can include modified amino acids, and it can be interrupted by non-amino acids. This term can also include amino acid polymer which has been modified (e.g., disulfide bond formation, glycosylation, lipidation, acetylation, phosphorylation or any other operations, such as conjugation with a labeling component). As used in the present disclosure, "miRNA" is non-encoded RNA of a small single chain molecule, generally consists of about 22 nucleotide sequences, and widely exists in plants, animals and some viruses. microRNA mainly conducts gene post-transcriptional regulation by RNA silencing, because the miRNAs play a role by complementary pairing with a mRNA base, a target mRNA molecule may be silenced by several means as follows: 1) cleavage of the mRNA into two different sequences; 2) decreasing the stability of the mRNA by shortening a tail of polyA of the mRNA; and 3) decreasing the binding of the mRNA with a ribosome to further decrease translation of the mRNA.

The miRNA widely exists in mammalian cells, and there are different high abundance expression of the miRNA among different tissues, such as up-regulated expression of miR-122 in live cells, up-regulated expression of miR-133, miR-206, and miR-208 in muscle cells, up-regulated expression of miR-17-92, and miR-126 in endothelial cells, up-regulated expression of miR-142-3p, miR-142-5p, miR-16, miR-21, miR-223, miR-24, miR-27, etc. in myelocyte, up-regulated expression of let-7, and miR-30c in adipocyte, up-regulated expression of miR-ld, and miR-149 in heart cells, up-regulated expression of miR-192, miR-194, and miR-204 in kidney cells, and up-regulated expression of let-7, miR-133, and miR-126 in lung epithelial cells.

As used in the present disclosure, the term "circular RNA" refers to a closed circular RNA molecule. In some embodiments, the circular RNA molecule in the present disclosure is formed by linking a 5' end at the upstream of a linear RNA molecule with a 3' end at the downstream of the linear RNA molecule to form a circular form. In some embodiments, the circular RNA molecule in the present disclosure is formed by a cleavage and cyclization reaction of a cyclization precursor RNA molecule to form a circular form.

As used in the present disclosure, the term "cyclization precursor RNA molecule" refers to an RNA precursor which is able to be cyclized to form the circular RNA, and it is generally formed by transcription of a DNA molecule of a cyclization precursor. In the present disclosure, the "cyclization precursor RNA molecule" can be used interchangeably with a "linear RNA molecule". In some embodiments, the cyclization precursor RNA includes elements as follows: a 5' homologous arm, a 3' intron, a second exon E2, a spacer region, an IRES element, a coding region, a spacer region, a first exon E1, a 5' intron, and a 3' homologous arm. In some embodiments, the 5' homologous arm, the 3' intron, the 5' intron, and the 3' homologous arm play a role in a process of formation of the circular RNA by cleaving the cyclization precursor RNA. Wherein, the intron has a ribozyme property, for example, by using the ribozyme property of the 5' intron, the connection between the 5' intron and the first exon E1 is broken under the trigger of GTP, the cleavage site can also further cause the connection between the 3' intron and the second exon E2 to be broken, and the second exon E2 is linked to the first exon E1 to form the circular RNA.

As used in the present disclosure, the term "IRES" is also known as an internal ribosome entry site, and "the internal ribosome entry site (IRES)" belongs to a translation control sequence, is usually located at a 5' end of a gene of interest, and translates RNA in a cap independent manner. The transcribed IRES can directly bind to ribosomal subunits, such that a mRNA initiation codon is properly orientated in the ribosome for translation. The IRES sequence is usually located in 5'UTR of mRNA (just upstream of the initiation codon). IRES functionally replaces the demand of various protein factors which interact with a translation mechanism of eukaryotes.

As used in the present disclosure, the term "coding region" refers to a gene sequence which can transcript a messenger RNA, and finally be translated to a target polypeptide or protein.

As used in the present disclosure, the term "expression" includes any step involved in polypeptide production, including but not limited to: transcription, post-transcriptional modification, translation, post-translational modification, and secretion.

As used in the present disclosure, the term "upstream" or "downstream" refers to the upstream and downstream along a protein translation direction of a coding region.

As used in the present disclosure, the term "complementary" or "hybridized" refers to "polynucleotide" and "oligonucleotide" related to base pairing rules (they are interchangeable terms, referring to a nucleotide sequence). For example, a sequence "CAGT" and a sequence "GTCA" are complementary. The complementation or hybridization can be "partial" or "entire". The "partial" complementation or hybridization refers to mispairing of one or more nucleic acid bases according to the base pairing rules, an "entire" or "complete" complementation or hybridization between nucleic acids means that each nucleic acid base matches with another base through Watson-Crick base pairs. The complementation or hybridization degree between nucleic acid chains has an important influence on the hybridization efficiency and intensity between the nucleic acid chains. This is especially important in an amplification reaction as well as a detection method which depends on binding between nucleic acids.

As used in the present disclosure, the terms "sequence identity" and "percent identity" refer to a percentage of nucleotides or amino acids which are same (i.e. identical) between two or more polynucleotides or polypeptides. A sequence identity between two or more polynucleotides or polypeptides can be measured by the method as follows: aligning nucleotide or amino acid sequences of the polynucleotides or polypeptides, and scoring the number of locations containing a same nucleotide or amino acid residue in the aligned polynucleotides or polypeptides, and comparing the number of the locations containing the same nucleotide or amino acid residue in the aligned polynucleotides or polypeptides with the number of locations containing different nucleotide or amino acid residues in aligned polynucleotides or polypeptides. The polynucleotide can be different at a location, for example, by containing different nucleotides (i.e., replacement or mutation) or deletion of nucleotide (i.e., nucleotide insertion or nucleotide deletion in one or two polynucleotides). The polypeptide can be different, for example, by containing different amino acids (i.e., replacement or mutation) or deletion of amino acid (i.e., amino acid insertion or amino acid deletion in one or two polypeptides). The sequence identity can be calculated by dividing the number of the locations containing the same nucleotide or amino acid residue by the total number of amino acid residues in the polynucleotide or polypeptide. For example, the percent identity can be calculated by dividing the number of the locations containing the same nucleotide or amino acid residue by the total number of the nucleotide or amino acid residues in the polynucleotide or polypeptide and multiplying by 100.

For example, when conducting comparison and alignment with maximum correspondence by using a sequence comparison algorithm or by visual inspection and measurement, two or more sequences or subsequences have at least 80%, 81%, 82%, 83%, 84%, 85%, 86%, 87%, 88%, 89%, 90%, 91%, 92%, 93%, 94%, 95%, 96%, 97%, 98% or 99% "sequence identity" or "percent identity" of nucleotide. In certain embodiments, the sequence is basically same in the entire length of any one or two compared biopolymers (e.g., polynucleotides).

As used in the present disclosure, the term "recombinant nucleic acid molecule" refers to a polynucleotide having sequences which are not linked together in nature. A recombinant polynucleotide can be included in a suitable vector, and this vector can be used for transformation to a suitable host cell. Then the polynucleotide is expressed in a recombinant host cell to produce, for example, "recombinant polypeptide", "recombinant protein", "fusion protein", etc.

As used in the present disclosure, the term "vector" refers to a DNA construct, it contains a DNA sequence which is operably linked to a suitable control sequence, so as to express a target gene in a suitable host.

As used in the present disclosure, the term "recombinant expression vector" refers to a DNA structure which is used for expressing, for example, a polynucleotide encoding a required polypeptide. The recombinant expression vector can include, for example, (i) a collection of genetic elements having a regulatory effect on gene expression, for example, promoters and enhancers; (ii) a structure or coding sequence which is transcribed into mRNA and translated into protein; and (iii) proper transcription subunits for transcription and translation initiation and terminator sequences. The recombinant expression vector is constructed in any suitable way. The property of the vector is not important, and any vectors can be used, including plasmid, virus, bacteriophage and transposon. The possible vector used in the present disclosure includes but is not limited to chromosome, non-chromosome and synthetic DNA sequences, for example, virus plasmid, bacterial plasmid, bacteriophage DNA, yeast plasmid and a vector derived from a combination of plasmid and bacteriophage DNA, and DNA from slow virus, retrovirus, cowpox, adenovirus, avian pox, baculovirus, SV40 and pseudorabies, etc.

As used in the present disclosure, the term "host cell" refers to a cell into which exogenous polynucleotide has been introduced, including filial generation of such cells. The host cell includes a "transformant" and a "transformed cell", which includes primary transformed cell and filial generation derived therefrom. The host cell is a cell system of any types which can be used for producing an antibody molecule of the present invention, including eukaryotic cells, for example, mammalian cells, insect cells, and yeast cells; and prokaryotic cells, for example, Escherichia coli cells. The host cell includes cultivated cells, and also includes cells within transgenic animals, transgenic plants or cultivated plant tissues or animal tissues. The term "recombinant host cell" covers host cells that are different from parental cells after the circular RNA molecule, the cyclization precursor RNA molecule, the recombinant nucleic acid molecule or the recombinant expression vector is introduced, and the recombinant host cell is specifically obtained by transformation. The host cells of the present disclosure can be prokaryotic cells or eukaryotic cells, as long as they are cells into which the circular RNA molecule, the cyclization precursor RNA molecule, the recombinant nucleic acid molecule or the recombinant expression vector of the present disclosure can be introduced.

As used in the present disclosure, the term "individual", "patient" or "subject" includes mammals. The mammals include but are not limited to domestic animals (e.g., cows, sheep, cats, dogs and horses), primates (e.g., human and non-human primates such as monkeys), rabbits and rodents (e.g., mice and rats).

As used in the present disclosure, the term "tumor" refers to growth and proliferation of all neoplastic cells, whether malignant or benign, and all pre-cancerous and cancerous cells and tissues. When mentioned herein, the term "cancer", "cancerous" and "tumor" are not mutually exclusive.

As used in the present disclosure, the term "pharmaceutically acceptable" refers to an excipient, composition and/or dosage form suitable for contact with human and animal tissues without excessive toxicity, irritation, anaphylaxis or other problems or complications, and commensurate with a reasonable benefit/risk ratio within the scope of reasonable medical judgment.

As used in the present disclosure, the term "target cell" refers to a cell type to which it is desired to delivery an exogenous molecule (e.g., a circular RNA molecule), or an exogenous molecule has specific high expression or specific low expression in cells. The target cells can be ex-vivo cells, or cells included in a subject's body. In some embodiments, the target cells are diseased cells, for example, tumor cells.

As used in the present disclosure, the term "target tissue" is a tissue including a target cell. In some embodiments, the target tissue is a diseased tissue, for example, a tumor tissue.

As used in the present disclosure, the term "non-targeted cell" refers to a cell type to which it is undesirable to delivered an exogenous molecule (e.g., a circular RNA molecule), or in which the expression of an exogenous molecule is opposite to that in target cells. In some embodiments, non-target cells are non-diseased cells, for example, normal tissue cells.

As used in the present disclosure, the term "non-target tissue" is a tissue which does not include target cells. In some embodiments, the non-target tissues are tissues without lesions, for example, various healthy tissues.

### Technical solution

In the technical solution of the present disclosure, the meanings represented by the numbers in the nucleotide and amino acid sequence table in the description are as follows:
a sequence shown in SEQ ID NO. 1 is a nucleotide sequence of 3'UTR control
a sequence show in SEQ ID NO. 2 is a nucleotide sequence of a miR-122 recognition region
a sequence shown in SEQ ID NO. 3 is a nucleotide sequence of a miR-152 recognition region
a sequence shown in SEQ ID NO. 4 is a nucleotide sequence of a miR-148 recognition region
a sequence shown in SEQ ID NO. 5 is a nucleotide sequence of a miR-194 recognition region
a sequence shown in SEQ ID NO. 6 is a nucleotide sequence of a miR-199 recognition region
a sequence shown in SEQ ID NO. 7 is a nucleotide sequence of a miR-215 recognition region
a sequence shown in SEQ ID NO. 8 is a nucleotide sequence of a miR-18 recognition region
a sequence shown in SEQ ID NO. 9 is a nucleotide sequence of a miR-20 recognition region
a sequence shown in SEQ ID NO. 10 is a nucleotide sequence of a miR-24 recognition region
a sequence shown in SEQ ID NO. 11 is a nucleotide sequence of a miR-30b recognition region
a sequence shown in SEQ ID NO. 12 is a nucleotide sequence of a miR-30c recognition region
a sequence shown in SEQ ID NO. 13 is a nucleotide sequence of a miR-32 recognition region
a sequence shown in SEQ ID NO. 14 is a nucleotide sequence of a miR-141 recognition region
a sequence shown in SEQ ID NO. 15 is a nucleotide sequence of a miR-192 recognition region
a sequence shown in SEQ ID NO. 16 is a nucleotide sequence of a miR-193 recognition region
a sequence shown in SEQ ID NO. 17 is a nucleotide sequence of a miR-194 recognition region
a sequence shown in SEQ ID NO. 18 is a nucleotide sequence of a miR-200b recognition region
a sequence shown in SEQ ID NO. 19 is a nucleotide sequence of a miR-204 recognition region
a sequence shown in SEQ ID NO. 20 is a nucleotide sequence of a miR-18 recognition region
a sequence shown in SEQ ID NO. 21 is a nucleotide sequence of a miR-19a recognition region
a sequence shown in SEQ ID NO. 22 is a nucleotide sequence of a miR-20 recognition region
a sequence shown in SEQ ID NO. 23 is a nucleotide sequence of a miR-24 recognition region
a sequence shown in SEQ ID NO. 24 is a nucleotide sequence of a miR-32 recognition region
a sequence shown in SEQ ID NO. 25 is a nucleotide sequence of a miR-126 recognition region
a sequence shown in SEQ ID NO. 26 is a nucleotide sequence of a miR-133 recognition region
a sequence shown in SEQ ID NO. 27 is a nucleotide sequence of a miR-141 recognition region
a sequence shown in SEQ ID NO. 28 is a nucleotide sequence of a miR-193 recognition region
a sequence shown in SEQ ID NO. 29 is a nucleotide sequence of a miR-200b recognition region
a sequence shown in SEQ ID NO. 30 is a nucleotide sequence of a miR-213 recognition region
a sequence shown in SEQ ID NO. 31 is a nucleotide sequence of a miR-1 recognition region
a sequence shown in SEQ ID NO. 32 is a nucleotide sequence of a miR-133 recognition region
a sequence shown in SEQ ID NO. 33 is a nucleotide sequence of a miR-143 recognition region
a sequence shown in SEQ ID NO. 34 is a nucleotide sequence of a miR-149 recognition region
a sequence shown in SEQ ID NO. 35 is a nucleotide sequence of a miR-206 recognition region
a sequence shown in SEQ ID NO. 36 is a nucleotide sequence of a miR-208 recognition region
a sequence shown in SEQ ID NO. 37 is a nucleotide sequence of a miR-7 recognition region
a sequence shown in SEQ ID NO. 38 is a nucleotide sequence of a miR-9 recognition region
a sequence shown in SEQ ID NO. 39 is a nucleotide sequence of a miR-124 recognition region
a sequence shown in SEQ ID NO. 40 is a nucleotide sequence of a miR-125 recognition region
a sequence shown in SEQ ID NO. 41 is a nucleotide sequence of a miR-128 recognition region
a sequence shown in SEQ ID NO. 42 is a nucleotide sequence of a miR-132 recognition region
a sequence shown in SEQ ID NO. 43 is a nucleotide sequence of a miR-135 recognition region
a sequence shown in SEQ ID NO. 44 is a nucleotide sequence of a miR-137 recognition region
a sequence shown in SEQ ID NO. 45 is a nucleotide sequence of a miR-139 recognition region
a sequence shown in SEQ ID NO. 46 is a nucleotide sequence of a miR-149 recognition region
a sequence shown in SEQ ID NO. 47 is a nucleotide sequence of a miR-153 recognition region
a sequence shown in SEQ ID NO. 48 is a nucleotide sequence of a miR-183 recognition region
a sequence shown in SEQ ID NO. 49 is a nucleotide sequence of a miR-190 recognition region
a sequence shown in SEQ ID NO. 50 is a nucleotide sequence of a miR-219 recognition region
a sequence shown in SEQ ID NO. 51 is a nucleotide sequence of a miR-16 recognition region
a sequence shown in SEQ ID NO. 52 is a nucleotide sequence of a miR-21 recognition region
a sequence shown in SEQ ID NO. 53 is a nucleotide sequence of a miR-24 recognition region
a sequence shown in SEQ ID NO. 54 is a nucleotide sequence of a miR-27 recognition region
a sequence shown in SEQ ID NO. 55 is a nucleotide sequence of a miR-99a recognition region
a sequence shown in SEQ ID NO. 56 is a nucleotide sequence of a miR-127 recognition region
a sequence shown in SEQ ID NO. 57 is a nucleotide sequence of a miR-132 recognition region
a sequence shown in SEQ ID NO. 58 is a nucleotide sequence of a miR-142 recognition region
a sequence shown in SEQ ID NO. 59 is a nucleotide sequence of a miR-151 recognition region
a sequence shown in SEQ ID NO. 60 is a nucleotide sequence of a miR-181 recognition region
a sequence shown in SEQ ID NO. 61 is a nucleotide sequence of a miR-189 recognition region
a sequence shown in SEQ ID NO. 62 is a nucleotide sequence of a miR-212 recognition region
a sequence shown in SEQ ID NO. 63 is a nucleotide sequence of a miR-223 recognition region
a sequence shown in SEQ ID NO. 64 is a nucleotide sequence of a miR-17 recognition region
a sequence shown in SEQ ID NO. 65 is a nucleotide sequence of a miR-92 recognition region
a sequence shown in SEQ ID NO. 66 is a nucleotide sequence of a miR-126 recognition region
a sequence shown in SEQ ID NO. 67 is a nucleotide sequence 3'UTR 1×miRNA-122
a sequence shown in SEQ ID NO. 68 is a nucleotide sequence of 3'UTR 3×miRNA-122 a sequence shown in SEQ ID NO. 69 is a nucleotide sequence of EV29-LUC-3UTR
a sequence shown in SEQ ID NO. 70 is a nucleotide sequence of EV29-LUC-3UTR+1×miR-122
a sequence shown in SEQ ID NO. 71 is a nucleotide sequence of EV29-LUC-3UTR+3xmiR-122
a sequence shown in SEQ ID NO. 72 is a nucleotide sequence of EV29-IL-2-3UTR
a sequence shown in SEQ ID NO. 73 is a nucleotide sequence of EV29-IL-2-3UTR+3×miR-122
a sequence shown in SEQ ID NO. 74 is a nucleotide sequence of EV29-CTLA-4(HC)-3UTR
a sequence shown in SEQ ID NO. 75 is a nucleotide sequence of EV29-CTLA-4(LC)-3UTR
a sequence shown in SEQ ID NO. 76 is a nucleotide sequence of EV29-CTLA-4(HC)-3UTR+3xmiR-122
a sequence shown in SEQ ID NO. 77 is a nucleotide sequence of EV29-CTLA-4(LC)-3UTR+3xmiR-122
a sequence shown in SEQ ID NO. 78 is a nucleotide sequence of CVB3 IRES
a sequence shown in SEQ ID NO. 79 is a nucleotide sequence of EV24 IRES
a sequence shown in SEQ ID NO. 80 is a nucleotide sequence of EV29 IRES
a sequence shown in SEQ ID NO. 81 is a nucleotide sequence of EV33 IRES
a sequence shown in SEQ ID NO. 82 is a nucleotide sequence of IRES chimeric with EV24 and CVB3
a sequence shown in SEQ ID NO. 83 is a nucleotide sequence of IRES chimeric with EV29 and CVB3
a sequence shown in SEQ ID NO. 84 is a nucleotide sequence of IRES chimeric with EV33 and CVB3
a sequence shown in SEQ ID NO. 85 is a nucleotide sequence of a 5' spacer region sequence 1
a sequence shown in SEQ ID NO. 86 is a nucleotide sequence of a 5' spacer region sequence 2
a sequence shown in SEQ ID NO. 87 is a nucleotide sequence of a 3' spacer region sequence 1
a sequence shown in SEQ ID NO. 88 is a nucleotide sequence of a 3' spacer region sequence 2
a sequence shown in SEQ ID NO. 89 is a nucleotide sequence of a 3' spacer region sequence 3
a sequence shown in SEQ ID NO. 90 is a nucleotide sequence of an exon element 1 (E1) of a class I PIE system
a sequence shown in SEQ ID NO. 91 is a nucleotide sequence of an exon element 2 (E2) of a class I PIE system
a sequence shown in SEQ ID NO. 92 is a nucleotide sequence of a 5' intron of a class I PIE system
a sequence shown in SEQ ID NO. 93 is a nucleotide sequence of a 3' intron of a class I PIE system
a sequence shown in SEQ ID NO. 94 is a nucleotide sequence of a 5' homologous arm sequence 1 (H1)
a sequence shown in SEQ ID NO. 95 is a nucleotide sequence of a 5' homologous arm sequence 2 (H2)
a sequence shown in SEQ ID NO. 96 is a nucleotide sequence of a 3' homologous arm sequence 1
a sequence shown in SEQ ID NO. 97 is a nucleotide sequence of a 3' homologous arm sequence 2

### Circular RNA molecule

The circular RNA molecule provided by the present disclosure includes a coding region which encodes a target polypeptide, and an expression regulatory element which is operably linked to the coding region; the expression regulatory element has characteristics shown in any one of the group consisting of (i)-(ii): (i) enhancing expression of the target polypeptide in target cells or target tissues, and (ii) attenuating expression of the target polypeptide in non-target cells or non-target tissues. The level of the target polypeptide expressed by the circular RNA molecule is regulated by using the expression regulatory element, so that he circular RNA molecule is specifically highly expressed in the target cells and the target tissues, and specifically lowly expressed in the non-target cells and non-target tissues, so as to realize differential expression of the circular RNA molecule in different cells and tissues, and provide targeted drug delivery in disease related cells and tissues with a safe and effective treatment means.

In some embodiments, the expression regulatory element in the circular RNA molecule attenuates expression of the target polypeptide in the non-target cells or the non-target tissues. Wherein the expression regulatory element includes one or more miRNA recognition regions which are hybridized with a miRNA sequence. The present disclosure first found that addition of a miRNA recognition region in the circular RNA molecule can specifically decrease a protein expression level of the circular RNA molecule in the non-target cells or non-target tissues without influencing a circular RNA cyclization efficiency, and first confirmed that the miRNA can be used to regulate the protein translation and expression level of the circular RNA molecule.

In the present disclosure, the miRNA recognition region is hybridized with the sequence of the miRNA, namely the miRNA recognition region has a reverse complementary sequence of the miRNA sequence. In some embodiments, the sequence of the miRNA recognition region is completely complementary with the sequence of the miRNA. In some additional embodiments, the sequence of the RNA recognition region and the sequence of the miRNA is partially complementary, namely, one or more mispaired or unpaired bases are allowed to exist between the sequence of the miRNA recognition region and the sequence of the miRNA. Specifically, provided that the miRNA recognition region can bind to the miRNA, the level of expression of the target polypeptide by the circular RNA molecule is inhibited.

In the present disclosure, the miRNA recognition region included in the expression regulatory element is one or more, for example, the expression regulatory element includes 1, 2, 3, 4, 5, 6, or 7 miRNA recognition regions. Further, when the number of miRNA recognition regions is two or more, the sequence of each miRNA recognition region and the sequence of other miRNA recognition regions can be the same or different, namely, the expression regulatory element can include multiple miRNA recognition regions which bind to a same type of miRNA, and also includes multiple miRNA recognition regions which bind to different types of miRNAs; and the number of the miRNA recognition regions which bind to the same type of miRNA can also be one or more.

In some preferred embodiments, the expression level of the miRNA hybridized with the miRNA recognition region in the non-target cells or non-target tissues is higher than the expression level of the miRNA in the target cells or target tissues. By using the differential expression of the miRNA between different cells and tissues, the expression of the circular RNA molecule in non-target cells or non-target tissues is prevented or reduced, but normal expression of the circular RNA molecule in the target cells and target tissues is maintained. When the circular RNA molecule is administered into the subject's body, its expression presents remarkable tissue specificity, by using tissue specific expression of the circular RNA molecule, targeted protein expression and drug delivery for a specific tissue and cell is achieved, and a target protein is not expressed in the non-target cell, organ, and tissue, effectively increasing the targetability and safety of drug delivery.

In some preferred embodiments, the target cells or target tissues are diseased cells or tissues, and the non-target cells or non-target tissues are non-diseased cells or tissues. The expression of the circular RNA molecule in the non-target cells or non-target tissues is inhibited, so as to avoid organ and tissue injury due to expression of the target polypeptide in healthy cells or tissues, having high clinical safety. In some more preferred embodiments, the target cells or target tissues are tumor cells or tumor tissues, the expression level of the miRNA in the non-tumor cells or non-tumor tissues is higher than the expression level of the miRNA in tumor cells or tumor tissues, while a high expression level of the circular RNA molecule in tumor cells or tumor tissues is maintained, the expression of the circular RNA molecule in healthy cells or tissues is inhibited or eliminated, increasing the specificity of delivering the target polypeptide to the tumor cells, and tissues by the circular RNA molecule, while realizing treatment of diseases such as tumors, effectively reducing adverse reaction and injury to the healthy tissues and organs, and having an important clinical application value for treatment of diseases such as tumors.

In some embodiments, the expression regulatory element only includes a miRNA recognition region hybridized with a miRNA sequence. In some other embodiments, the expression regulatory element can also include other nucleotide sequences, for example, a UTR sequence, etc. When the expression regulatory element includes other nucleotide sequences, the miRNA recognition region can be located within the expression regulatory element, at a 5' end or at a 3' end. For example, the miRNA recognition region is 1, 2, 3, etc. sequence regions located within a 3' UTR sequence which are invertedly complementary with the miRNA sequence. Further, when the number of miRNA recognition regions is two or more, any two of the miRNA recognition regions can be directly linked, and they can also be inserted in the 3' UTR sequence at intervals.

In some embodiments, the expression regulatory element includes one or more miRNA recognition regions which are hybridized with sequences of one or more miRNAs as follows:
miR-122, miR-152, miR-148, miR-194, miR-199, miR-215, miR-18, miR-20, miR-24, miR-30b, miR-30c, miR-32, miR-141, miR-192, miR-193, miR-194, miR-200b, miR-204, miR-18, miR-19a, miR-20, miR-24, miR-32, miR-126, miR-133, miR-141, miR-193, miR-200b, miR-213, miR-1, miR-133, miR-143, miR-149, miR-206, miR-208, miR-7, miR-9, miR-124, miR-125, miR-128, miR-132, miR-135, miR-137, miR-139, miR-149, miR-153, miR-183, miR-190, miR-219, miR-16, miR-21, miR-24, miR-27, miR-99a, miR-127, miR-132, miR-142, miR-151, miR-181, miR-189, miR-212, miR-223, miR-17, and miR-92;
In some embodiments, the miRNA recognition region provided by the present disclosure is selected from any one of (a₁)-(a₂):
(a₁) a nucleotide sequence is a sequence shown in any one of SEQ ID NOs: 2-66, and
(a₂) a nucleotide sequence is a sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity with the sequence shown in any one of SEQ ID NOs: 2-66, and capable of binding to miRNA to regulate the expression of the target polypeptide.

In some embodiments, the expression regulatory element provided by the present disclosure includes one or more miRNA recognition regions shown in (a₁) and (a₂). For example, the expression regulatory element includes 1, 2, 3, 4, 5, 6, 7, etc. miRNA recognition regions shown in (a₁) and (a₂).

The miRNA recognition region in the above-mentioned expression regulatory element can bind to the miRNA which is expressed with high abundance in liver cells/tissues, kidney cells/tissues, lung cells/tissues, myocardial cells/tissues, nerve cells/ tissues, human hematopoietic stem cells/tissues, and endothelial cells/tissues, the expression of the circular RNA molecule in the healthy cells and tissues is inhibited, avoiding the injury to the non-diseased organs and tissues. Meanwhile, in the diseased cells and tissues (e.g., tumor cells or tumor tissues), the expression abundance of related miRNA is relatively low, which does not influence efficient expression of the target polypeptide by the circular RNA molecule in tumor cells and tissues, realizing treatment of diseases such as tumors.

In some preferred embodiments, the nucleotide sequence of the expression regulatory element is that one or more miRNA recognition regions shown in (a₁) or (a₂) are inserted into the nucleotide sequence shown in SEQ ID NO: 1. For example, the nucleotide sequence of the expression regulatory element is that 1, 2, 3, 4, etc. miRNA recognition regions shown in (a₁) or (a₂) are inserted into the nucleotide sequence shown in SEQ ID NO: 1. The insertion number and insertion location of the miRNA recognition region can be determined according to actual needs.

In some specific embodiments, the insertion number of the miRNA recognition region is one, and its insertion location is between 88th and 89th nucleotides, between 48th and 49th nucleotides, or between 9th and 10th nucleotides of the nucleotide sequence shown in SEQ ID NO: 1. In some additional specific embodiments, the insertion number of the miRNA recognition region is 3, and the miRNA recognition regions are simultaneously inserted between 88th and 89th nucleotides, between 48th and 49th nucleotides, and between 9th and 10th nucleotides of the nucleotide sequence shown in SEQ ID NO: 1 to obtain a target expression regulatory element. In some more specific embodiments, the miRNA recognition regions are simultaneously inserted between 88th and 89th nucleotides, between 48th and 49th nucleotides, and between 9th and 10th nucleotides of the nucleotide sequence shown in SEQ ID NO: 1, and hybridized with a same type of miRNA.

For example, in the present disclosure, explanation is carried out by means of the expression regulatory element including a miR-122 recognition region, when the expression regulatory element includes the miR-122 recognition region of the sequence shown in SEQ ID NO. 2, the sequence of the expression regulatory element can be any one of the following:
1) a nucleotide sequence shown in SEQ ID NO. 67 which is obtained by inserting one sequence shown in SEQ ID NO. 2 between 88th and 89th nucleotides of the sequence shown in SEQ ID NO: 1;
2) a nucleotide sequence shown in SEQ ID NO. 68, which is obtained by inserting one sequence shown in SEQ ID NO. 2 between 88th and 89th nucleotides, between 48th and 49th nucleotides, and between 9th and 10th nucleotides of the sequence shown in SEQ ID NO: 1.

For the expression regulatory element of the miRNA recognition region including the sequences shown in SEQ ID NOs: 3-66, its expression regulatory element can be obtained by any one of the above 1) or 2), which will not be exemplified one by one in the present disclosure.

In some embodiments, the expression regulatory element includes a sequence, which has at least 80%, 81%, 82%, 83%, 84%, 85%, 86%, 87%, 88%, 89%, 90%, 91%, 92%, 93%, 94%, 95%, 96%, 97%, 98%, 99%, or 100% sequence identity with the sequence shown in any one of SEQ ID NOs: 2-68, and can realize hybridization of the miRNA recognition region with the miRNA.

Production of the miRNA in cells generally has the following procedures: pri-miRNA is treated with a Drosha enzyme within a nucleus to form Precursor miRNAs (pre-miRNAs) with a stem-loop structure of about 70nt, and these pre-miRNAs are transported outside the cell nucleus, then treated with cytosolic Dicer enzyme, and digested to form mature miRNAs. A mature miRNA nomenclature recognized in the art usually cover the following contents:
1) an arm of pre-miRNA from which the mature miRNA is derived is designated, "5p" means that the miRNA is from a 5' arm of pre-miRNA hairpin, and "3p" means that the miRNA is from a 3'arm of pre-miRNA hairpin. In the present disclosure, unless specially designated by a 3p or 5p name, miR mentioned by a number can refer to any one of two types of miRNAs originated from opposite arms of the same pre-miRNA (e.g., miR-122 represents any one or a combination of miR-122-5p, and miR-122-3p).
2) miRNAs are almost all in a unique coding order, but for miRNA having several different bases, it may be marked with a letter for showing, for example, miR-124a and miR-124b. In the present disclosure, unless specially designated by names such as a, b, c, etc., the miR mentioned by a number can refer to any one of several types of miRNAs having high degree of homology.
3) If the mature miRNAs are the same, but the pre-miRNA and the pri-miRNA and genes encoding them come from different genomes, they are represented with numbers, for example, miR-194-1 and miR-194-2 mean that two mature miRNAs after cleavage of pre-miRNA, and pri-miRNA are completely same, but are of two different origins.

In some specific embodiments, the expression regulatory element which attenuates the expression of the target polypeptide in the non-target cells or non-target tissues shown in (ii) is linked at the downstream of the coding region.

In some embodiments, the circular RNA molecule also includes a translation initiation element which is operably linked to the coding region. In the present disclosure, the translation initiation element may be any sequence element which is able to recruit ribosome, and initiate the translation process of the circular RNA molecule. For example, the translation initiation element is an IRES element, a m6A modification sequence, or an initiation sequence of rolling circle translation and the like.

In some specific embodiments, the translation initiation element is an IRES element, and the IRES element is linked at the upstream of the coding region, and is able to effectively initiate the translation and expression of the target polypeptide in the coding region.

In some preferred embodiments, the IRES sequence includes a sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% sequence identity with the nucleotide sequence of one or more sequences selected from the group consisting of sequences shown in SEQ ID NOs: 78-81. In some embodiments, the IRES sequence is CVB3 IRES of the nucleotide sequence shown in SEQ ID NO: 78, EV24 IRES of the nucleotide sequence shown in SEQ ID NO: 79, EV29 IRES of the nucleotide sequence shown in SEQ ID NO: 80, or EV33 IRES of the nucleotide sequence shown in SEQ ID NO: 81. In some embodiments, the IRES sequence includes a chimera sequence of CVB3v IRES with any one of EV24 IRES, EV29 IRES and EV33 IRES. In some embodiments, the IRES sequence includes a chimera sequence of CVB3v with EV24 IRES, and its nucleotide sequence is as shown in SEQ ID NO: 82. In some embodiments, the IRES sequence includes a chimera sequence of CVB3v with EV29 IRES, and its nucleotide sequence is as shown in SEQ ID NO: 83. In some embodiments, the IRES sequence includes a chimera sequence of CVB3v with EV33 IRES, and its nucleotide sequence is as shown in SEQ ID NO: 84. The IRES sequence of the present disclosure is able to realize efficient and lasting expression of the target polypeptide in the target cells and target tissues, increasing the efficiency of expressing the target polypeptide by the circular RNA molecule in the target cells and target tissues.

In some embodiments, the circular RNA molecule also includes one or more elements as follows: a 5' spacer region, a 3' spacer region, a second exon, a first exon, and an expression regulatory element shown in (i). By setting one or more other expression elements, the circular RNA molecule can be obtained by multiple cyclization methods, and the tissue specificity and the expression efficiency of the target polypeptide expressed by the circular RNA molecule are further increased.

In some embodiments, the circular RNA molecule includes a 5' spacer region located at the upstream of the translation initiation element, and a 3' spacer region located at the downstream of the expression regulatory element. In some preferred embodiments, the 5' spacer region sequence is a sequence which has at least 80%, 81%, 82%, 83%, 84%, 85%, 86%, 87%, 88%, 89%, 90%, 91%, 92%, 93%, 94%, 95%, 96%, 97%, 98%, 99%, or 100% sequence identity with a nucleotide sequence shown in any one of SEQ ID NOs: 85-86. In some preferred embodiments, the 3' spacer region sequence is a sequence which has at least 80%, 81%, 82%, 83%, 84%, 85%, 86%, 87%, 88%, 89%, 90%, 91%, 92%, 93%, 94%, 95%, 96%, 97%, 98%, 99%, or 100% sequence identity with a nucleotide sequence shown in any one of SEQ ID NOs: 87-89.

In some embodiments, the circular RNA molecule also includes a second exon located at the upstream of the 5' spacer region, and a first exon located at the downstream of the 3' spacer region. In some preferred embodiments, the second exon sequence is a sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% sequence identity with the nucleotide sequence shown in SEQ ID NO: 91. In some preferred embodiments, the first exon sequence is a sequence which has at least 80%, 81%, 82%, 83%, 84%, 85%, 86%, 87%, 88%, 89%, 90%, 91%, 92%, 93%, 94%, 95%, 96%, 97%, 98%, 99%, or 100% sequence identity with the nucleotide sequence shown in SEQ ID NO: 90.

In some embodiments, the circular RNA molecule also includes an expression regulatory element which enhances the expression of the target polypeptide in the target cells or target tissues shown in (i), for example, the expression regulatory element is a promoter having tissue specificity, a kozak sequence used for increasing the translation level of the encoding region, and the like.

In some preferred embodiments, the circular RNA molecule includes the following elements which are sequentially linked: a second exon, a 5' spacer region, an IRES element, a coding region, an expression regulatory element, a 3' spacer region and a first exon.

In the present disclosure, by the selected 5' spacer region, 3' spacer region, expression regulatory element, IRES element, second exon and first exon, specific high expression of the circular RNA molecule in the target tissues and target cells can be realized, and specific low expression of the circular RNA molecule in the non-target cells and non-target tissues, providing clinical treatment of diseases such as tumors with a safe and effective treatment strategy.

In some embodiments, the target polypeptide encoded by the circular RNA molecule is a polypeptide having an activity of disease prevention or treatment. The circular RNA molecule can specifically express the target polypeptide in the diseased tissues and cells, to realize targeted treatment of diseases, and decrease the toxic and side effects due to expression of the target polypeptide in healthy cells and tissues.

In some preferred embodiments, the target polypeptide is a polypeptide having an activity of tumor prevention or treatment, in the present disclosure, the polypeptide having a tumor immunotherapy activity can be any types of polypeptides having the tumor immunotherapy activity, and the sequence of the polypeptide having the tumor immunotherapy activity is not specifically limited in the present disclosure.

In some embodiments, the polypeptide having the tumor immunotherapy activity is cytokine. For example, the cytokine includes but is not limited to interleukin (IL), interferon (IFN), tumor necrosis factor (TNF), granulocyte-macrophage colony-stimulating factor (GM-CSF), and macrophage colony-stimulating factor (M-CSF). The circular RNA molecule, by specifically expressing cytokine in tumor cells and tumor tissues, enriches the cytokine in the tumor tissues, which is more beneficial to stimulate the body's antitumor immune response, effectively killing the tumor cells, avoiding high expression of the cell molecule in healthy tissues and organs, and reducing occurrence probability of an adverse reaction.

In some embodiments, the polypeptide having the tumor immunotherapy activity is an antigen-binding fragment. More particularly, the polypeptide having the tumor immunotherapy activity is an antigen-binding fragment which specifically binds to a tumor antigen. For example, the tumor antigen includes but is not limited to CD19, CD20, CD22, CD30, CD33, CD38, CD123, CD138, CD171, AFP, CEA, PSCA, GD2, NKG2D, BCMA, EGFR, Her2, EGFRvIII , CD171, FAP, IL13Rα2, VEGFR1, VEGFR2, GPC-3, Mesothelin, claudin 18.2, EpCAM, MUC1, MUC16, EPHA2, EPHA3, CD133, and PSMA. The circular RNA molecule, by specifically expressing the antigen-binding fragment which specifically binds to the tumor antigen in the tumor cells and tumor tissues, blocks a tumor growth factor signaling pathway, and plays a role in killing the tumor cells.

In the present disclosure, the antigen-binding fragment includes an antibody which specifically binds to a tumor antigen, or an antigen which includes a part of a complete antibody and binds to the complete antibody.

Further, the antigen-binding fragment includes a natural antibody and an artificial antibody which cover various structures, including but not limited to a monoclonal antibody, a polyclonal antibody, a polyspecific antibody (e.g., a bispecific antibody), a single chain antibody (e.g., scFv), a complete antibody, such as Fv, Fab, Fab', Fab'-SH, and F(ab')₂, a linear antibody, a single-domain antibody, a bivalent or bispecific antibody or a fragment thereof, a camelidae antibody, and a bispecific antibody or polyspecific antibody formed from an antibody fragment.

In some embodiments, the circular RNA molecule includes a nucleotide sequence shown in SEQ ID NO: 73, or a sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% sequence identity with the nucleotide sequence shown in SEQ ID NO: 73. Cytokine IL-2 is expressed in a coding region of a circular RNA molecule shown in SEQ ID NO: 73, and its expression regulatory element includes 3 miR-122 recognition regions; compared with a circular RNA molecule without the expression regulatory element, the content of IL-2 expressed by the circular RNA molecule shown in SEQ ID NO: xx in liver cells is significantly decreased, whereas in tumor cells, the expression content of IL-2 is not significantly changed; after the two types of circular RNA molecules are delivered into tumor model mice, the level of IL-2 expressed by the circular RNA molecule shown in SEQ ID NO: 73 in tumor is increased, and the content of IL-2 in plasma is significantly deceased, which indicates that the circular RNA molecule with the expression regulatory element is able to realize specific high expression in the tumor cells and tissues, and specific low expression in healthy cells and tissues such as livers.

In some additional embodiments, the circular RNA molecule is a nucleotide molecule which is obtained by simultaneously replacing 1380-1401th, 1441-1462th, and 1503-1524th nucleotide sequences of the sequence shown in SEQ ID NO: 73 with a sequence shown in any one of SEQ ID NOs: 2-66.

In some embodiments, the circular RNA molecule includes the nucleotide sequence shown in SEQ ID NO: xx, or a sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% sequence identity with the nucleotide sequence shown in SEQ ID NO: xx. An immune checkpoint inhibitor CTLA is expressed in a coding region of a circular RNA molecule shown in any one of SEQ ID NOs: 76-77, and its expression regulatory element includes three miR-122 recognition regions; after being delivered into a tumor mouse model, compared with the circular RNA molecule without the expression regulatory element, the content of CTLA expressed by the circular RNA molecule shown in any one of SEQ ID NOs: 76-77 in liver cells is significantly decreased, whereas in tumor cells, the expression content of CTLA is not significantly changed; and after the two types of circular RNA molecules are delivered into tumor model mice, the level of CTLA expressed by the circular RNA molecule shown in any one of SEQ ID NOs: 76-77 in the tumor is increased, the content of CTLA in plasma is significantly decreased, which indicates that the circular RNA molecule with the expression regulatory element is able to realize the specific high expression in the tumor cells, and specific low expression in healthy cells such as liver cells, etc.

In some additional embodiments, the circular RNA molecule is a nucleotide molecule which is obtained by simultaneously replacing 2217-2238th, 2278-2299th, and 2340-2361th nucleotide sequences of the sequence shown in SEQ ID NO: 76 with a sequence shown in any one of SEQ ID NOs: 3-66.

In some additional embodiments, the circular RNA molecule is a nucleotide molecule which is obtained by simultaneously replacing 1518-1539th, 1579-1600th, and 1641-1662th nucleotide sequences of the sequence shown in SEQ ID NO: 77 with a sequence shown in any one of SEQ ID NOs: 3-66.

### Cyclization precursor RNA molecule

The cyclization precursor RNA molecule provided by the present disclosure is able to be cyclized to form the above-mentioned circular RNA molecule. The cyclization precursor RNA molecule can be cyclized by multiple methods, and in some embodiments, the cyclization precursor RNA molecule is ligated by T4 ligase (T4 DNA ligase, T4 RNA ligase I, T4 RNA ligase II) to form the circular RNA molecule in the present disclosure. In some additional embodiments, the cyclization precursor RNA molecule is cyclized via cleavage and ligation by a PIE system to form the circular RNA molecule of the present disclosure.

In some preferred embodiments, the cyclization precursor RNA molecule also includes a 5' homologous arm located at the upstream of the second exon, and the 5' homologous arm sequence is a sequence which has at least 80%, 81%, 82%, 83%, 84%, 85%, 86%, 87%, 88%, 89%, 90%, 91%, 92%, 93%, 94%, 95%, 96%, 97%, 98%, 99%, or 100% sequence identity with the nucleotide sequence shown in any one of SEQ ID NOs: 94-95.

In some preferred embodiments, the cyclization precursor RNA molecule also includes a 3' homologous arm located at the downstream of the first exon. The 3' homologous arm sequence is a sequence which has at least 80%, 81%, 82%, 83%, 84%, 85%, 86%, 87%, 88%, 89%, 90%, 91%, 92%, 93%, 94%, 95%, 96%, 97%, 98%, 99%, or 100% sequence identity with the nucleotide sequence shown in any one of SEQ ID NOs: 96-97.

In some preferred embodiments, the cyclization precursor RNA molecule also includes a 3' intron located between the second exon and the 5' homologous arm, and the 3' intron is a sequence which has at least 80%, 81%, 82%, 83%, 84%, 85%, 86%, 87%, 88%, 89%, 90%, 91%, 92%, 93%, 94%, 95%, 96%, 97%, 98%, 99%, or 100% sequence identity with the nucleotide sequence shown in SEQ ID NO: 93.

In some preferred embodiments, the cyclization precursor RNA molecule also includes a 5' intron located between the first exon and the 3' homologous arm, and the 5'intron is a sequence which has at least 80%, 81%, 82%, 83%, 84%, 85%, 86%, 87%, 88%, 89%, 90%, 91%, 92%, 93%, 94%, 95%, 96%, 97%, 98%, 99%, or 100% sequence identity with the nucleotide sequence shown in SEQ ID NO: 92.

In some specific embodiments, the cyclization precursor RNA molecule includes the following elements which are sequentially linked:
a 5' homologous arm, a 3' intron, a second exon, a 5' spacer region, an IRES element, a coding region, an expression regulatory element, a 3' spacer region, a first exon, a 5' intron and a 3' homologous arm.

The cyclization precursor RNA molecule is cyclized via the following procedures: by using the ribozyme characteristic of the intron, under the trigger of GTP, the junction of the 5' intron and the first exon is broken; a ribozyme cleavage site of the first exon further attacks the junction the junction of 3' intron and the second exon to be broken, the 3' intron is dissociated, and the first exon is linked to the second exon to form the circular RNA molecule.

In some embodiments, the present disclosure provides a recombinant nucleic acid molecule, which can be transcribed to form the above cyclization precursor RNA molecule. In order to enable the recombinant nucleic acid molecule to be further transcribed to form the RNA molecule, the recombinant nucleic acid molecule can also include a regulatory sequence. For example, the regulatory sequence is a T7 promoter linked at the upstream of the 5' homologous arm.

In some embodiments, the present disclosure provides a recombinant expression vector, which includes the above recombinant nucleic acid molecule. Wherein the vector linking the recombinant nucleic acid molecule can be various vectors commonly used in the art, for example, a pUC57 plasmid, etc. Further, the recombinant nucleic acid molecule includes a restriction enzyme cutting site, so that the recombinant expression vector is digested to obtain a linearized vector suitable for transcription.

In some embodiments, the present disclosure provides a recombinant host cell, including at least one of the circular RNA molecule, the cyclization precursor RNA molecule, the recombinant nucleic acid molecule and the recombinant expression vector.

### Pharmaceutical preparation or composition

The circular RNA molecule, the cyclization precursor RNA molecule, the recombinant nucleic acid molecule, the recombinant expression vector or the recombinant host cell in the present disclosure can be mixed with one or more pharmaceutically acceptable carriers to be made into any clinically administrable dosage forms. For example, the pharmaceutical preparation is a tablet, capsule, injection, spray, granule, powder, suppository, pill, cream, paste, gel, powder, oral solution, inhalant, suspension or dry suspension, etc.

In some embodiments, the circular RNA molecule is encapsulated in a liposome material, and made into a pharmaceutical preparation. For example, the liposome material includes a liposome polymer (lipopolyplex, LPR), a cationic liposome complex (lipoplex, LP), lipid nanoparticles (LNPs), etc. Encapsulating the circular RNA molecule using the liposome material can further increase targetability and delivery efficiency of drug delivery, so as to realize specific expression in target cells and target tissues.

It is well known in the art that the liposome material, as a drug delivery carrier, when delivering a drug molecule such as a small molecule, an antibody, and a protein to a subject's body, exhibits advantages such as targeting, sustained or controlled release, good biocompatibility and low toxic and side effects. However, researches have found that when a nucleic acid molecule of mRNA is delivered by the liposome material, the mRNA molecule universally exhibits a trend of high abundance expression in liver cells. Especially, whether the intratumoral delivery or other in-vivo drug administration ways, the liposome material encapsulated mRNA molecules are all likely to be enriched in liver cells, and expresses the target polypeptide with high abundance in the liver cells, causing strong hepatotoxicity.

The circular RNA molecule in the present disclosure, under a condition of being delivered via the liposome material, highly expresses the target polypeptide only in the target cells and the target tissues, and lowly expresses the target polypeptide in the non-target cells and the non-target tissues, exhibiting a trend of tissue specific expression, thereby effectively solving the problem of high abundance expression in the liver cells at present during delivery of the mRNA molecule which expresses the target polypeptide via the liposome material, causing the strong hepatotoxicity.

In some embodiments, the pharmaceutical preparation or composition in the present disclosure which is formed by encapsulating the circular RNA molecule with the liposome material, is suitable to targetedly deliver a therapeutic target polypeptide into the tumor cells and tissues, and the circular RNA molecule is lowly expressed or not expressed in cells, tissues and organs such as livers, which has high tumor targeting, and can avoid toxic and side effects due to high abundance expression in liver.

Further, the pharmaceutical preparation or composition in the present disclosure is suitable to be used as an injection, and administered into a subject's body in an intratumor injection way, highly expresses the target polypeptide such as cytokine, an antibody, and an immune checkpoint inhibitor in the tumor locally, having advantages such as high tumor targeting, significant immunotherapeutic effects, and low toxic and side effects.

### Medical application

The present disclosure provides application of the circular RNA molecule, the cyclization precursor RNA molecule, the recombinant nucleic acid molecule, , the recombinant expression vector or the recombinant host cell in preparation of a drug for preventing or treating diseases. By using the tissue specific expression of the circular RNA molecule, targeted delivery of the target polypeptide having disease prevention or treatment effects into diseased cells and tissues is realized.

In some preferred embodiments, the circular RNA molecule, etc. are used in preparation of a drug for preventing or treating tumors, more preferably preparation of an intratumoral injection for preventing or treating tumors. After a drug prepared from the circular RNA molecule is administered into the subject's body, the target polypeptides such as cytokine, an antigen-binding fragment, and an immune checkpoint inhibitor expressed by the circular RNA molecule are highly enriched in tumor tissues, and have low expression or no expression in healthy tissues and organs, having high clinical medicinal safety and tumor treatment effects.

In some embodiments, the present disclosure provides a method for preventing or treating diseases, including administering the circular RNA molecule, the cyclization precursor RNA molecule, the recombinant nucleic acid molecule, the recombinant expression vector, the recombinant host cell, or the pharmaceutical preparation or composition to a subject. The circular RNA molecule is suitable to be delivered to the subject's body, realizing targeted treatment for diseased cells, tissues, and organs.

Optionally, the administration is selected from oral administration, intraperitoneal administration, intravenous administration, intra-arterial administration, intramuscular administration, intradermal administration, subcutaneous administration, transdermal administration, nasal administration, transrectal administration, intratumoral injection, intraluminal retention of tumor, nerve intrathecal injection, intrathecal injection or systemic administration; preferably intratumoral injection.

### Examples

Other objectives, features and advantages of the present disclosure will become obvious from the following detailed description. But it should be understood that the detailed description and specific examples (while showing the specific embodiments of the present disclosure) are given only for the explanatory purpose, because after reading the detailed description, various changes and modifications made within the spirit and scope of the present disclosure will become obvious to those skilled in the art.

Unless otherwise specified, the experimental techniques and experimental methods used in this example are all conventional technical methods, for example, the experimental methods in which specific conditions are not specified in the following examples are usually in accordance with conventional conditions such as the conditions described in Sambrook *et al., Molecular Cloning: Laboratory manual* (New York: Cold Spring Harbor Laboratory Press, 1989), or the conditions recommended by the manufacturer. Unless otherwise specified, the materials and reagents used in the examples can all be obtained from formal commercial channels.

### Example 1: Preparation of circular mRNA containing a miR-122 recognition region and its expression in A549 cells

### (1) Construction of plasmid

A Luciferase target gene containing different sequences was constructed, and this step was entrusted to Suzhou Genewiz Biotechnology Co., Ltd for gene synthesis and cloning.

The obtained gene fragment was linked to a pUC57 vector. The following plasmids were obtained:
pUC57-EV29-LUC,
pUC57-EV29-LUC-3'UTR,
pUC57-EV29-LUC-3'UTR+1 xmiR-122.

### (2) Preparation of linear plasmid template

### 1) Extraction of plasmid

①Puncture bacteria synthesized externally was activated at 37°C and 220 rpm for 3-4h.
② The activated bacterial solution was taken for amplification culture at 37°C and 220 rpm overnight.
③ A plasmid was extracted (a Tiangen endotoxin-free small amount Midiprep Kit), and an OD value was measured.

### 2) Digestion of plasmid

The plasmid prepared in the above 1) was digested by a Xbal single digestion method.

A digestion system was as follow:

**Table 1**

| Reagent | Volume |
|---|---|
| plasmid | 10 µg |
| Enzyme (1000 units) | 5 µl |
| 10×cutsmart buffer | 50 µl |
| Nuclease free, H₂O | Total 500 µl |

Enzymatic digestion was conducted at 37°C overnight. The enzyme-digested products were recycled by using a general DNA Gel Extraction Kit (Tiangen Biochemical Technology Co., Ltd), and an OD value was measured and the enzyme-digested products were identified by using 1% agarose gel electrophoresis. The purified linear plasmid template was used for in-vitro transcription.

### (3) Preparation of linear mRNA by in-vitro transcription

### 1) In-vitro transcription

### mRNA was synthesized by using a T7 in-vitro transcription kit (APE×BIO T7 High Yield RNA Synthesis Kit)

A transcription system was as follows:

**Table 2**

| Reagent | Volume |
|---|---|
| 10×Reaction Buffer | 2 µl |
| ATP (20 mM) | 2 µl |
| CTP (20 mM) | 2 µl |
| UTP (20 mM) | 2 µl |
| GTP (20 mM) | 2 µl |
| linearized DNA template | 1 µg |
| T7 RNA Polymerase Mix | 2 µl |
| RNA nuclease free, H₂O | Total 20 µl |

Incubation was carried out at 37°C for 2.5 hours, and then the linear DNA template was digested with DNase I. Digestion condition: digestion was carried out at 37°C for 15 minutes.

### 2) Purification of the linear mRNA

The transcribed product obtained in the above 1) was purified by using a silica spin column method (Thermo, GeneJET RNA Purification Kit), an OD value was measured and an RNA size was identified by 1% denatured agarose gel electrophoresis.

A formula of 1% denatured agarose gel was as follows:
1 g of agarose was weighted to 72 ml of nuclease-free H₂O, and heated to be dissolved in a microwave oven; and
when the above agarose was cooled to 55~60°C, 0.1% gel red, 10 ml of 10×MOPS, 18 ml of formaldehyde were added in a fume hood, then a gel was filled.

The process of denatured agarose gel electrophoresis is as follows: a same volume of a sample RNA and 2×Loading buffer were taken, and denatured at 65~70°C for 5-10 min. The sample was loaded, electrophoresis was conducted under a condition of 100V/30 min, and then photos were taken by a gel imaging system.

### (4) cyclization of mRNA

1) cyclization reagent:
GTP Buffer: 50 mM Tris-HCI, 10 mM MgCl₂, 1 mM DTT, pH~7.5.
2) Cyclization system and condition:

**Table 3**

| Solution | Volume |
|---|---|
| mRNA | 25 µg mRNA |
| GTP solution (20 mM) | 50 µl |
| GTP buffer | made up to 500 µl |

The above solution was heated at 55°C for 15 minutes, then placed onto ice, the cyclized RNA product was purified by a silica spin column method (Thermo, GeneJET RNA Purification Kit), an OD value was measured and a DNA size was identified by 1% denatured agarose gel electrophoresis.
3) Identification of circular RNA by 1% denatured agarose gel
A. reagent preparation: 1 g agarose powder was added into 72 ml of nuclease-free water, and heated to thaw the agarose, and 10 ml of 10×MOPS buffer was added. Then 18 ml of fresh 37% formaldehyde was added in a fume hood, well mixing was performed, a gel was poured into a slot.
B. detection of mRNA: about 500 ng of mRNA solution was taken, the same volume of 2×RNA loading buffer was added, uniform mixing was performed, the mixture was heated at 65°C for 5 minutes, and agarose gel detection was conducted.

### (5) Transfection of circular mRNA encoding Luciferase to A549 cells and measurement of fluorescence intensity

1) Cell culture:
A549 was inoculated in a DMEM high glucose medium containing 10% fetal calf serum and 1% double-antibody, and incubated at 37°C in a 5% CO₂ incubator. The cells were subcultured every other 2-3 days.
2) Cell transfection: Before the transfection, A549 cells were inoculated in a 24-well plate at 1×10⁵/well, and incubated at 37°C in a 5% CO₂ incubator. After the cells reached a confluence of 70-90%, by using a Lipofectamine Messenger Max (Invitrogen) transfection reagent, the mRNA was transfected to A549 cells in an amount of 500 ng/well, and the specific operation was as follows:
① Dilution of Messenger MAX^{™} Reagent

**Table 4**

| Reagent | Volume/well |
|---|---|
| MEM serum-free medium | 25 µl |
| Messenger MAX^{™} Reagent | 0.75 µl |

After dilution and mixing, incubation was carried out by standing at room temperature for 10 min.
② Dilution of mRNA

**Table 5**

| Reagent | Volume/well |
|---|---|
| mRNA | 1µg |
| MEM serum-free medium | made up to 25µl |

③ Mixed and diluted Messenger MAX^{™} Reagent and mRNA (1:1) was taken

**Table 6**

| Reagent | Volume/well |
|---|---|
| Diluted Messenger MAX^{™} Reagent | 25 µl |
| Diluted mRNA | 25 µl |

After dilution and mixing, incubation was carried out by standing at room temperature for 5min.
④ 50 µl of the above mixed liquor was sucked and slowly added into the 24-well plate in an adherent manner, and incubation was carried out at 37°C in the 5% CO₂ incubator.
2) Detection of protein expression
① Collection and counting of cells: after the cells were transfected for 24 h, the cells were digested by using trypsin (Gibco), and centrifuged at 1000rpm for 5 minutes, the supernatant was abandoned, the cells were resuspended in PBS, a trypan blue solution was mixed with the cell suspension at 1:1, and then the cells were counted using a Countstar cell counter.
② Cell lysis: in each transfection group, 2×10⁴ cells were taken, the cells were centrifuged, then the supernatant was abandoned, 20 µl of cell lysis solution was added, and lysis was carried out on ice for 5 minutes.
③ Detection of luciferase protein expression by a microplate reader: the lysate was placed in a 96-well white plate, 100 µl of luciferase reaction substrate was added into each well, and a luminescent value was detected by the microplate reader.

Experimental results:

**Table 7**

| Plasmid extraction | Concentration | Volume | |
|---|---|---|---|
| PUC-EV29-LUC | 347.6 ng/µl | 240 µl | about 83.4 µg |
| PUC-EV29-LUC-3UTR | 186.9 ng/µl | 240 µl | about 44.8 µg |
| PUC-EV29-LUC-3UTR+1 × miR-122 | 181.2 ng/µl | 240 µl | about 43.5 µg |

| Products after digestion | | | |
|---|---|---|---|
| PUC-EV29-LUC | 209.3 ng/µl | 240 µl | about 50 µg, yield: about 60.0% |
| PUC-EV29-LUC-3UTR | 231.1 ng/µl | 120 µl | about 27 µg, yield: about 60.3% |
| PUC-EV29-LUC-3UTR+1×miR-122 | 234.2 ng/µl | 120 µl | about 28 µg, yield: about 64.3% |

| Transcribed products | | | |
|---|---|---|---|
| EV29-LUC | 1008.9 ng/µl | 900 µl | about 908 µg |
| EV29-LUC-3UTR | 1244.4 ng/µl | 900 µl | about 1.1mg |
| EV29-LUC-3UTR+1 xmiR-122 | 1178.4 ng/µl | 900 µl | about 940µg |

| Cyclized products | | | |
|---|---|---|---|
| EV29-LUC | 1897.5 ng/µl | 450 µl | about 853.8 µg, yield: about 94.8% |
| EV29-LUC-3UTR | 1572.3 ng/µl | 450 µl | about 707.5 µg, yield: about 78.6% |
| EV29-LUC-3UTR+1 xmiR-122 | 1741.2 ng/µl | 450 µl | about 783.5 µg, yield: about 87.0% |

As shown by the results in Fig. 1 and Fig. 2, based on the molecule design of the circular mRNA with an addition of the 1×miR-122 recognition region, digestion and linearization of its DNA template, RNA in-vitro transcription and cyclization of mRNA all can be normally conducted, which indicates that addition of 1×miR-122 recognition region has no influence on the normal preparation process of the circular mRNA. The cell experimental results (Fig. 3) indicate that the circular mRNA with the addition of the 1×miR-122 recognition region can effectively express Luciferase protein encoded by the circular mRNA in A549 cells. Compared with the wild type EV29-Luc circular mRNA in the control group and EV29-Luc-3'UTR in the control group, the expression quantity of Luciferase by EV29-Luc-3'UTR-1×miR-122 is slightly low, and the possible reason is that there is a background level of miR-122 expression in A549 cells.

### Example 2: Expression of circular mRNA containing multiple miR-122 recognition regions in A549 cells

On the basis of the above Example 1, more miR-122 recognition regions were added based on the circular mRNA EV29-LUC-3'UTR+1×miR-122, for example 3×miR-122 was added. The methods of plasmid DNA linearization, circular precursor mRNA in-vitro transcription, purification of the circular precursor mRNA, a cyclization reaction of the mRNA, purification of the circular mRNA, the cell culture and Luciferase detection are all the same as those in 1.1 of Example 1.

Steps of cell transfection: Before transfection, A549 cells were inoculated in a 24-well plate at 1×10⁵/well, and incubated at 37°C in a 5% CO₂ incubator. After the cells reached a confluence of 70-90%, the mRNA was transfected to A549 cells in an amount of 500 ng/well by using a Lipofectamine Messenger Max (Invitrogen) transfection reagent, and the specific operation was as follows:
① Dilution of Messenger MAX^{™} Reagent

**Table 8**

| Reagent | Volume/well |
|---|---|
| MEM serum-free medium | 25 µl |
| Messenger MAX^{™} Reagent | 0.75 µl |

After dilution and mixing, incubation was carried out by standing at room temperature for 10 minutes.
② Dilution of mRNA

**Table 9**

| Reagent | Volume/well |
|---|---|
| Circular RNA mRNA | 500 ng |
| miR-122/NC miRNA | 125 ng |
| MEM serum-free | made up to 25 µl |
| medium | |

After dilution and mixing, incubation was carried out by standing at room temperature for 5 minutes.
③ 50 µl of the above mixed liquor was sucked and slowly added into the 24-well plate in an adherent manner, and incubation was carried out at 37°C in the 5% CO₂ incubator. The experimental results show that (Fig. 4) compared with the treatment group transfected by NC miRNA, the treatment group transfected by miR-122 showed significant Luciferase expression inhibition, whether circular mRNA of 1×miR-122 or circular mRNA of 3×miR-122, its expression quantity of Luciferase was decreased to a background level. Wherein, an expression inhibition effect of circular mRNA of 3xmiR-122 is significant. This experimental result indicates that the circular mRNA into which the miR-122 recognition region was introduced was significantly disturbed by the miR-122 within the cells, showing evident translation inhibition effects. Wherein in the NC group, Luciferase expression of pUC-EV29-LUC-3'UTR+1×miR-122 and pUC-EV29-LUC-3'UTR+3xmiR-122 also showed some degree of decrease, and its reason is that there was a background level of miR-122 expression in the A549 cells.

### Example 3: Influence of circular mRNA including different tissue specific miRNA recognition regions on expression of Luciferase in specific tissue cells

On the basis of the above Example 1, EV29-Luc circular RNA containing different micoRNA sequences was constructed, and its expression among different cell lines was analyzed. Wherein the methods of plasmid DNA linearization, in-vitro transcription of the circular precursor mRNA, purification of the circular precursor mRNA, a cyclization reaction of the mRNA, purification of the circular mRNA, cell transfection and Luciferase detection are all the same as those in 1.1 of Example 1.

### 1) Resuscitation and subculturing of MRC-5 and IMR-90 human lung fibroblast strain

MRC-5 and IMR-90 human lung fibroblast strains were taken out from a liquid nitrogen storage tank, and a cryogenic vial was rapidly put in a water bath of 37°C, and shaken rapidly until a cryopreservation solution in the cryogenic vial was completely thawed. The cell concentration was adjusted, the cells were inoculated in a MEM medium containing 10% fetal calf serum and 1% double-antibody, and then the inoculated cells were incubated at 37°C in a CO₂ incubator. After cell adherent growth was observed to be up to 70% of a bottom area of a culture dish, the old culture solution was abandoned, washing was performed with 4 ml of PBS twice, and 1 ml pancreatin was sucked up and added thereto. The cells were put in a 37°C incubator for about 2 min, cell digestion was observed with an inverted microscope, after the digestion degree was appropriate, 1-2 ml of medium was taken and added into the cells, and the cells were pipetted up and down until all cells fall from the bottom of the culture dish. A suitable amount of medium was taken and added into a new culture dish, a cell suspension was added, observation was carried out under a microscope, well shaking was performed, and incubation was carried out at 37°C. Passage was carried out once every 2-3 days.

### 2) Resuscitation and subculturing of LO2 and Chang liver human normal liver cell strain

LO2 and Chang liver human normal liver cell strains were taken out from a liquid nitrogen storage tank, and a cryogenic vial was rapidly put into a water bath of 37°C, and shaken rapidly until a cryopreservation solution in the cryogenic vial was completely thawed. The cell concentration was adjusted, the cells were inoculated in a DMEM medium containing 10% fetal calf serum and 1% double-antibody, and then the inoculated cells were incubated at 37°C in a CO₂ incubator. A cell passage method was the same as above.

### 3) Resuscitation and subculturing of H9C2 rat cardiomyocyte strain

An H9C2 rat cardiomyocyte strain was taken out from a liquid nitrogen storage tank, and a cryogenic vial was rapidly put in a water bath of 37°C, and shaken rapidly until a cryopreservation solution in the cryogenic vial was completely thawed. The cell concentration was adjusted, the cells were inoculated in a DMEM medium containing 1% fetal calf serum, and then the inoculated cells were incubated at 37°C in a CO₂ incubator. A cell passage method was the same as above.

### 4) Primary culture of rat spinal cord neurons and astrocytes

### a. Cultivation of rat spinal cord neurons

A full term new-born rat was sterilized, and then placed in a plate, a spinal cord was taken out under a supine position, meninges were peeled off, and a spinal cord tissue was broken, and digested at 37°C with 0.25% trypsin for 15minutes, then pancreatin action was terminated with a DMEM culture solution containing 10% fetal calf serum, filtration was performed, pipetting up and down was performed, then centrifugation was performed at 220xg/min, the supernatant was abandoned, and a cell pellet in a lower layer was re-suspended with a culture solution containing fetal calf serum. The above-mentioned cell suspension was put in an incubator of 37°C to be incubated, and transferred to an incubator of 37°C after inoculation, then the medium was changed to a serum-free medium (Neurobasal + 2%B27 + 0.5 mM glutamine +1% double-antibody), and incubation was carried out. On day 3 of cell culture, cytarabine with a concentration 10 µmol/L was added, after 24 hours, the cell culture solution was changed, after 3 days, half amount of the cell culture solution was changed once, and all the experiments began 5 to 7 days after cell seeding.

### b. Culture and purification of astrocytes:

An encephalon was taken out from a full term new-burn rat according to the above method, then cut to pieces, digested with pancreatin, filtered and centrifuged, the cells were inoculated in a cell culture plate or dish coated with polylysine (a medium is a DMEM culture solution containing 10% fetal calf serum), and incubated at 37°C in a 5% CO₂ cell incubator, after 24 hours, the culture solution was changed, non-adherent cells with poor viability or dead cells were removed, and then the culture solution was changed once every 3 days. Human primary astrocytes were inoculated in the cell culture plate or dish coated with polylysine in a ratio of 1:3, and incubated at 37° in a 2% CO₂ incubator, and passage was performed in accordance with this method for 4 to 5 generations.

### 5) Resuscitation and subculturing of HSPC-1 human hematopoietic stem cell strain

A HSPC-1 human hematopoietic stem cell strain was taken out from a Liquid nitrogen storage tank, and a cryogenic vial was rapidly put in a water bath of 37°C, and quickly shaken until a cryopreservation solution in the cryogenic vial was completely thawed. The cell concentration was adjusted, the cells were inoculated in a RPMI-1640 medium containing 10% fetal calf serum and 1% double-antibody, then the inoculated cell were incubated at 37°C in a CO₂ incubator. A cell passage method was the same as above.

### 6) Resuscitation and subculturing of HUV-EC-C human umbilical vein endothelial cell strain

**1.1 A HUV-EC-C human umbilical vein endothelial cell strain was taken from a liquid nitrogen storage tank, and a cryogenic vial was rapidly put in a water bath of 37°C, and shaken rapidly until a cryopreservation solution in the cryogenic vial was completely thawed. The cell concentration was adjusted, the cells were inoculated in a F-12K medium containing 10% fetal calf serum and 1% double-antibody, and then the incubated cells were incubated at 37°C in a CO₂ incubator. A cell passage method was the same as above.**

### 7) Resuscitation and subculturing of HK-2 human renal cortical cell strain

A HK-2 human renal cortical cell strain was taken out from a liquid nitrogen storage tank, and a cryogenic vial was rapidly put in a water bath of 37°C, and shaken quickly until a cryopreservation solution in the cryogenic vial was completely thawed. The cell concentration was adjusted, the cells were inoculated in a Keratinocyte-SFM medium containing 5 ng/mL rhEGF, and then the inoculated cells were incubated at 37°C in a CO₂ incubator. A cell passage method was the same as above.

Based on the circular RNA molecule of the sequence shown in SEQ ID NO. 70, influence of replacement of one of the following mi RNA recognition regions in the expression regulatory element of the circular RNA on inhibition of circular RNA expression:

**Table 10**

| High abundance expression of microRNA in liver cells | | |
|---|---|---|
| Name of microRNA | Inhibition ratio in LO2 | Inhibition ratio in Chang |
| miR-122 | 70% | 78% |
| miR-148 | 65% | 60% |
| miR-152 | 45% | 62% |
| miR-194 | 53% | 44% |
| miR-199 | 46% | 62% |
| miR-215 | 58% | 49% |

**Table 11**

| High abundance expression of microRNA in kidney cells | |
|---|---|
| Name of microRNA | Inhibition ratio in HK-2 |
| miR-18 | 65% |
| miR-20 | 75% |
| miR-24 | 66% |
| miR-30b | 62% |
| miR-30c | 52% |
| miR-32 | 45% |
| miR-141 | 69% |
| miR-192 | 55% |
| miR-193 | 45% |
| miR-194 | 50% |
| miR-200b | 65% |
| miR-204 | 66% |

**Table 12**

| High abundance expression of microRNA in lung cells | | |
|---|---|---|
| Name of microRNA | Inhibition ratio in MRC-5 | Inhibition ratio in IMR-90 |
| miR-18 | 60% | 49% |
| miR-19a | 69% | 82% |
| miR-20 | 55% | 63% |
| miR-24 | 57% | 68% |
| miR-32 | 45% | 42% |
| miR-126 | 64% | 51% |
| miR-133 | 56% | 68% |
| miR-141 | 51% | 78% |
| miR-193 | 36% | 50% |
| miR-200b | 60% | 62% |
| miR-213 | 55% | 46% |

**Table 13**

| High abundance expression of microRNA in cardiomyocytes | |
|---|---|
| Name of microRNA | H9C2 rat card iomyocytes |
| miR-1b | 78% |
| miR-1d | 65% |
| miR-133 | 55% |
| miR-143 | 47% |
| miR-149 | 60% |
| miR-206 | 55% |
| miR-208 | 75% |

**Table 14**

| High abundance expression of microRNA in nerve cells | | |
|---|---|---|
| Name of microRNA | Rat spinal cord neurons | Astrocytes |
| miR-7 | 81% | 70% |
| miR-9 | 55% | 85% |
| miR-124 | 78% | 69% |
| miR-125 | 62% | 62% |
| miR-128 | 66% | 84% |
| miR-132 | 78% | 45% |
| miR-135 | 70% | 50% |
| miR-137 | 81% | 68% |
| miR-139 | 64% | 66% |
| miR-149 | 59% | 69% |
| miR-153 | 52% | 75% |
| miR-183 | 45% | 55% |
| miR-190 | 76% | 79% |
| miR-219 | 72% | 80% |

**Table 15**

| High abundance expression of microRNA in HSPC-1 human hematopoietic stem cells | |
|---|---|
| Name of microRNA | HSPC-1 human hematopoietic stem cells |
| miR-16 | 55% |
| miR-21 | 45% |
| miR-24 | 78% |
| miR-27 | 85% |
| miR-99a | 72% |
| miR-127 | 66% |
| miR-132 | 66% |
| miR-142 | 80% |
| miR-151 | 67% |
| miR-181 | 52% |
| miR-189 | 69% |
| miR-212 | 45% |
| miR-223 | 78% |

**Table 16**

| High abundance expression of microRNA in endotheliocytes | |
|---|---|
| Name of microRNA | H9C2 rat card iomyocytes |
| miR-17 | 71% |
| miR-92 | 56% |
| miR-126 | 62% |

### Example 4: Expression of luciferase mediated by circular mRNA including a miR-122 recognition region in specific sites of mice

The circular mRNAs EV29-LUC-3'UTR, EV29-LUC-3'UTR+1×miR-122, and EV29-LUC-3'UTR+3×miR-122 in the above Example 1 and Example 2 were entrapped by a cationic liposome LNP delivery system. A circular RNA-lipid nanoparticle complex was prepared by microfluidic equipment, and mRNA active ingredients in a water phase were well mixed with four types of lipids in an organic phase to form a nano-sized mRNA product with high encapsulation efficiency. Firstly, a circular mRNA stock solution was diluted to 0.4 mg/mL with a citric acid solution of pH 4.0, four types of lipids were weighed and dissolved in an ethanol solution, and the total concentration of the lipid was 24.4 mg/mL. The two phases were rapidly mixed by using the microfluidic equipment, wherein a total flow rate was set to 12 mL/min, and a ratio of the water phase (the circular mRNA) to the organic phase (lipid) (v/v)=3:1. After the preparation was finished, ethanol was removed by means of dialysis or a tangential flow, and meanwhile the solution was replaced with a PBS solution of pH 7.4 to obtain the circular mRNA-lipid nanoparticle complex. The particle size and polydispersity index (PDI) of the circular mRNA-lipid nanoparticle complex were detected by using a dynamic light scatterer (DLS). The encapsulation efficiency of the circular mRNA in the complex was detected by using Ribogreen. The mice were administered in a way of intramuscular injection, and after 6 hours, expression of Luciferase in mice was determined.

The specific method was as follows: pUC-EV29-LUC-3UTR, pUC-EV29-LUC-3UTR+1×miR-122, and pUC-EV29-LUC-3UTR+3×miR-122 immunized mice were taken, intraperitoneally injected with 0.3 ml of luciferase substrate VivoGlo luciferin (in vivo Grade, Promega), and imaged after 8 minutes, and the in-vivo distribution and fluorescent expression intensity were observed.

Experimental results: as shown in Fig. 5, the luciferase of the mice in the control group (EV29-luc-3UTR) was mainly expressed in an intramuscular injection site and the liver; and the circular mRNA with addition of a single miR-122 recognition region (EV29-luc-3UTR+1×miR-122) was mainly expressed in the intramuscular injection site, and a small amount of expression appeared in the livers of individual mice; and for the circular mRNA with addition of three miR-122 recognition regions (EV29-luc-3UTR+3xmiR-122), the mRNA was expressed only in the intramuscular injection site, and expression in the liver was not detected. Thus it can be seen that introduction of miR-122 into the circular mRNA can effectively avoid expression of the circular mRNA in the liver, and the more the introduced miR-122 recognition regions, the more significant its inhibition effect of expression in the liver.

### Example 5: Test of Luciferase expression mediated by circular mRNA including a miR-122 recognition region in mouse tumor in situ and liver under intratumoral injection conditions

The circular mRNAs EV29-LUC-3'UTR, EV29-LUC-3'UTR+1 xmiR-122, and EV29-LUC-3UTR+3×miR-122 in the above Example 4 were entrapped by a cationic liposome LNP delivery system. The method was identical to that in Example 4. A construction method of a MC38 tumor model was as follows: before the study, female C57BL/6 (Beijing Vital River Laboratory Animal Technology Co., Ltd.) mice aged 6-8 weeks, with a body weight of 18-22 grams, were adapted in a feeding room (Genepharma) for at least three days.

The mice can be freely accessible to food and sterile water. Breeding environment: 12h-light 12h-dark day/night cycle was maintained, the humidity was kept at 40-70% and daily humidity difference was less than 5%; and the temperature was maintained at 20~25°C. Humanistic care was given in accordance with a 3R principle in experimental animal use. MC38 cells (from Nanjing COBIOER Bioscience Co., Ltd.) were cultivated in a Dulbecco's Modified Eagle's Medium (DMEM) high glucose medium, and 10% FBS (Fatal Bovine Serum) and 1% PS (penicillin-streptomycin solution) were added. The DMEM high glucose medium and FBS were purchased from Biological Industries, and the PBS was purchased from HyClone. During cell counting, after a Trypan blue solution (purchased from SIGMA) was mixed with a cell suspension (1:1), the cells were counted by using a Countstar cell counter. When the cells were collected, the cells were digested by using 0.25% trypsin-EDTA (Gibco), and re-suspended in a phosphate buffer with addition of Matrigel (Corning) (PBS, Shanghai BasalMedia Technologies Co., Ltd.), and each female C57BL/6J mouse was subcutaneously inoculated with 1×10⁶/150 µl of MC38 cells under the right axilla. Intratumoral injection of MC38 fluid was conduced by using a micro-syringe, and 10 µg of LNP entrapped circular mRNA was injected into each tumor. After 6 hours, expression of Luciferase in the mice was measured, and the specific operation steps were identical to those in Example 4.

As shown by the experimental results in Fig. 6 and Fig. 7, in the EV29-LUC-3UTR group, Luciferse was mainly expressed in the tumor and liver, and in the groups of EV29-LUC-3'UTR+1×miR-122, and EV29-LUC-3'UTR+3×miR-122, Luciferase was expressed only in tumor, and not expressed in the liver. The experimental evidence adequately shows that introduction of miR-122 in the circular mRNA can effectively avoid expression of Luciferse in the liver, so as to limit the expression site within the tumor.

### Example 6: Test of cytokine IL-2 expression mediated by the circular mRNA including a miR-122 recognition region in Hela cells and liver cells, and mouse tumor in situ and plasma under intratumoral injection conditions

The preparation method of the circular mRNAs EV29-IL-2-3UTR and EV29-IL-2-3UTR+3×miR-122 was identical to the method in Example 1. Two types of different circular mRNAs were transfected to HeLa cells and liver cells in an amount of 500 ng/well by using a Lipofectamine Messenger Max (Invitrogen) transfection reagent. The experimental results indicate that 12 hours after transfection to the HeLa cells, the expression quantities of the circular mRNAs EV29-IL-2-3UTR and EV29-IL-2-3UTR+3×miR-122 were 2520.5 ng/mL and 2074.3 ng/mL, respectively; 12 hours after transfection to the liver cells, IL-2 expression quantities were 226.8 ng/mL and 23.2 ng/mL, respectively. Thus it can be seen that the protein expression of EV29-IL-2-3UTR+3×miR-122 in liver cells was significantly inhibited.

**Table 17**

| | HeLa cells (ng/mL) | Chang Liver cells (ng/mL) |
|---|---|---|
| EV29-IL-2-3'UTR | 2520.5 | 226.8 |
| EV29-IL-2-3'UTR+3xmiR-122 | 2074.3 | 23.2 |

An entrapment method of the above circular mRNA by using a cationic liposome LNP delivery system was identical to the method in Example 4. A preparation method of a C57BL/6J mouse tumor model of MC38 tumor was the same as that in Example 5. The above circular mRNA-LNP complex was intratumorally injected to C57BL/6J mice with MC38 tumor, and the method was the same as that in Example 5. The mice were sacrificed 12 hours after the intratumoral injection, the content of IL-2 within the tumor and the content of IL-2 in peripheral blood were respectively measured by using an IL-12 ELISA test kit (Thermo Fisher).

Experimental results indicate that: 12 hours after the intratumoral injection of the circular mRNA EV29-IL-2-3'UTR, the content of IL-2 in the tumor was 146.5 ng/g, and the content of IL-2 in plasma was 153.2 ng/mL; and 12 hours after the intratumoral injection of the circular mRNA EV29-IL-2-3'UTR+3xmiR-122, the content of IL-2 in the tumor was 231.4 ng/mL, and the content of IL-2 in plasma was 16.5 ng/mL. Thus it can be seen that the IL-2 protein expressed by the circular mRNA EV29-IL-2-3UTR+3xmiR-122 were mainly distributed in the tumor, and the proteins expressed by EV29-IL-2-3UTR were respectively distributed in tumor and plasma, and the content of IL-2 expressed in the tumor is low. And the IL-2 protein in the plasma is mainly expressed via the liver and secreted to the blood circulation. Secretion of cytokine IL-23 into a circulatory system may lead to remarkable systemic toxicity. Therefore, intratumoral injection was conducted by using the circular mRNA EV29-IL-2-3UTR+3×miR-122, so as to avoid the mRNA being expressed in the liver and secreted into blood circulation, which is a safe and efficient strategy.

**Table 18**

| | Expression in tumor (ng/g) | Expression in plasma (ng/mL) |
|---|---|---|
| EV29-IL-2-3'UTR | 146.5 | 153.2 |
| EV29-IL-2-3'UTR+3xmiR-122 | 231.4 | 16.5 |

### Example 7: Test of anti-CTLA-4 monoclonal antibody expression mediated by circular mRNA including a miR-122 recognition region in Hela cells and liver cells, and mouse tumor in situ and plasma under intratumoral injection conditions

The preparation method of the circular mRNAs EV29-CTLA-4(HC)-3'UTR/EV29-CTLA-4(LC)-3'UTR and EV29-CTLA-4(HC)-3'UTR+3xmiR-122/EV29-CTLA-4(LC)-3'UTR+3×miR-122 was the same as the method in Example 1. By using a Lipofectamine Messenger Max (Invitrogen) transfection reagent, two types of different circular mRNA mixtures of HC and LC (1:1) were transfected to HeLa cells and liver cells in an amount of 500 ng/well. Experimental results indicate that 12 hours after transfection to the HeLa cells, the expression quantities of the circular mRNAs EV29-CTLA-4 (HC/LC)-3'UTR and EV29-CTLA-4(HC/LC)-3'UTR+3×miR-122 were 3631.5 ng/mL and 3307.2 ng/mL, respectively; 12 hours after transfection to the liver cells, the expression quantities of IL-2 were 463.2 ng/mL and 37.2 ng/mL, respectively. Thus it can be seen that the protein expression of EV29-IL-2-3'UTR+3×miR-122 in liver cells was inhibited significantly.

**Table 19**

| | HeLa cells (ng/mL) | Chang liver cells (ng/mL) |
|---|---|---|
| EV29-CTLA-4(HC/LC)-3'UTR | 3631.5 | 463.2 |
| EV29-CTLA-4(HC/LC)-3'UTR+3×miR-122 | 3307.2 | 37.2 |

An entrapment method for the above circular mRNA by using a cationic liposome LNP delivery system was identical to the method in Example 4. A preparation method of a C57BL/6J mouse tumor model of MC38 tumor was the same as that in Example 5. The above circular mRNA-LNP complex was intratumorally injected to C57BL/6J mice with MC38 tumor, and the method was the same as that in Example 5. 12 hours after the intratumoral injection, the mice were sacrificed, the content of the CTLA-4 monoclonal antibody within the tumor and the content of the CTLA-4 monoclonal antibody in peripheral blood were respectively measured by using a CTLA-4 antibody ELISA test kit (Thermo Fisher).

Experimental results indicate that 12 hours after the intratumoral injection of the circular mRNA EV29-CTLA-4(HC/LC)-3'UTR, the content of the CTLA-4 monoclonal antibody in the tumor was 283.8 ng/g, and the content of the CTLA-4 monoclonal antibody in plasma was 235.9 ng/mL; and 12 hours after the intratumoral injection of the circular mRNA EV29-CTLA-4(HC/LC)+3xmiR-122, the content of the CTLA-4 monoclonal antibody in the tumor was 473.4 ng/mL, and the content of the CTLA-4 monoclonal antibody in plasma was 31.6 ng/mL. Thus it can be seen that the IL-2 protein expressed by the circular RNA EV29-CTLA-4(HC/LC)+3 ×miR-122 were mainly distributed in the tumor, and the protein expressed by EV29-CTLA-4(HC/LC)-3'UTR were respectively distributed in the tumor and plasma, and the content of the CTLA-4 monoclonal antibody expressed in the tumor is low. And the CTLA-4 monoclonal antibody protein in the plasma was mainly expressed via the liver and secreted to the blood circulation. The CTLA-4 monoclonal antibody distributed within the tumor mainly acts on the effector T cells which were infiltrated into the tumor, compared with the CTLA-4 monoclonal antibody in a circulatory system, it is more beneficial to play a role in its T cell activation effect. Therefore, the intratumoral injection using the circular mRNA EV29-CTLA-4(HC/LC)-3'UTR+3×miR-122 can produce relatively high concentration of antibody concentrated within the tumor, which is more beneficial to trigger anti-tumor immunity, being an efficient strategy.

**Table 20**

| | Expression in tumor (ng/g) | Expression in plasma (ng/mL) |
|---|---|---|
| EV29-CTLA-4(HC/LC)-3'UTR | 283.8 | 235.9 |
| EV29-CTLA-4(HC/LC)-3'UTR+3xmiR-122 | 473.4 | 31.6 |

The above examples of the present disclosure are only the examples for clearly illustrating the present disclosure, rather than restriction on the embodiments of the present disclosure. Those of ordinary skill in the art, on the basis of the above illustration, can also make other different forms of changes or variations. All embodiments need not and cannot be exhaustive here. Any modifications, equivalent replacements and improvements made within the spirit and principle of the present disclosure are all included in the protection scope of the claims of the present disclosure.

## Claims

1. A circular RNA molecule, wherein the circular RNA molecule comprises a coding region which encodes a target polypeptide, and an expression regulatory element which is operably linked to the coding region; the expression regulatory element has characteristics shown in any one of the group consisting of (i)-(ii):
(i) enhancing expression of the target polypeptide in target cells or target tissues, and
(ii) attenuating expression of the target polypeptide in non-target cells or non-target tissues.

2. The circular RNA molecule according to claim 1, wherein the circular RNA molecule comprises an expression regulatory element as shown in (ii); wherein the expression regulatory element comprises one or more miRNA recognition regions which are hybridized with a sequence of miRNA; preferably, an expression level of the miRNA in the non-target cells or non-target tissues is higher than an expression level of the miRNA in the target cells or target tissues, more preferably, an expression level of the miRNA in non-tumor cells or non-tumor tissues is higher than an expression level of the miRNA in tumor cells or tumor tissues.

3. The circular RNA molecule according to claim 1 or 2, wherein the expression regulatory element comprises one or more miRNA recognition regions which are hybridized with sequences of one or more miRNAs as follows:
miR-122, miR-152, miR-148, miR-194, miR-199, miR-215, miR-18, miR-20, miR-24, miR-30b, miR-30c, miR-32, miR-141, miR-192, miR-193, miR-194, miR-200b, miR-204, miR-18, miR-19a, miR-20, miR-24, miR-32, miR-126, miR-133, miR-141, miR-193, miR-200b, miR-213, miR-1, miR-133, miR-143, miR-149, miR-206, miR-208, miR-7, miR-9, miR-124, miR-125, miR-128, miR-132, miR-135, miR-137, miR-139, miR-149, miR-153, miR-183, miR-190, miR-219, miR-16, miR-21, miR-24, miR-27, miR-99a, miR-127, miR-132, miR-142, miR-151, miR-181, miR-189, miR-212, miR-223, miR-17, and miR-92;
preferably, the miRNA recognition region comprises a sequence shown in any one of (a₁)-(a₂):
(a₁) is a nucleotide sequence shown in any one of SEQ ID NOs: 2-66;
(a₂) is a sequence having at least 90%, optionally at least 95%, preferably at least 97%, more preferably at least 98%, most preferably at least 99% sequence identity with the nucleotide sequence shown in (a₁);
optionally, the expression regulatory element comprises one or more miRNA recognition regions shown in any one of (a₁) or (a₂); preferably, the expression regulatory element is selected from a sequence in which one or more miRNA recognition regions shown in any one of (a₁) or (a₂) are inserted into a nucleotide sequence shown in SEQ ID NO: 1; preferably, a nucleotide sequence of the expression regulatory element is a sequence in which 1, 2 or 3 miRNA recognition regions shown in any one of (a₁) or (a₂) are inserted into the nucleotide sequence shown in SEQ ID NO: 1; more preferably, the miRNA recognition region is inserted between 88th and 89th nucleotides, between 48th and 49th nucleotides, or between 9th and 10th nucleotides of the nucleotide sequence shown in SEQ ID NO: 1.

4. The circular RNA molecule according to any one of claims 1-3, wherein the circular RNA molecule comprises a translation initiation element which is operably linked to the coding region, and an expression regulatory element shown in (ii); optionally, the translation initiation element is located at the upstream of the coding region, and the expression regulatory element shown in (ii) is located at the downstream of the coding region; preferably, the translation initiation element is an internal ribosome entry site (IRES) element;
preferably, the IRES element comprises any one of the following group of (iii)-(vi):(iii) a nucleotide sequence comprising one or more sequences selected from SEQ ID NOs: 78-81 in any combination;
(iv) a nucleotide sequence comprising a reverse complementary sequence of any sequence shown in SEQ ID NOs: 78-81;
(v) a reverse complementary sequence of a sequence which is capable of being hybridized with the nucleotide sequence shown in (i) or (ii) under highly stringent hybridization conditions or extremely highly stringent hybridization conditions; and
(vi) a sequence having at least 90%, optionally at least 95%, preferably at least 97%, more preferably at least 98%, most preferably at least 99% sequence identity with the nucleotide sequence shown in (iii) or (iv);
preferably, the IRES element is selected from any one of (b₁)-(b₇):
(b₁) a nucleotide sequence comprising a sequence shown in SEQ ID NO: 78;
(b₂) a nucleotide sequence comprising a sequence shown in SEQ ID NO: 79;
(b₃) a nucleotide sequence comprising a sequence shown in SEQ ID NO: 80;
(b₄) a nucleotide sequence comprising a sequence shown in SEQ ID NO: 81;
(b₅) a nucleotide sequence comprising a sequence shown in SEQ ID NO: 82;
(b₆) a nucleotide sequence comprising a sequence shown in SEQ ID NO: 83; and
(b₇) a nucleotide sequence comprising a sequence shown in SEQ ID NO: 84.

5. The circular RNA molecule according to claim 4, wherein the circular RNA molecule further comprises one or more elements as follows: a 5' spacer region, a 3' spacer region, a second exon, a first exon, and an expression regulatory element shown in (i);
preferably, wherein the circular RNA molecule comprises a 5' spacer region located at the upstream of the translation initiation element, and a 3' spacer region located at the downstream of the expression regulatory element;
more preferably, the 5' spacer region comprises a sequence shown in any one of (c₁)-(c₂):
(c₁) a nucleotide sequence shown in any one of SEQ ID NOs: 85-86;
(c₂) a sequence having at least 90%, optionally at least 95%, preferably at least 97%, more preferably at least 98%, most preferably at least 99% sequence identity with the nucleotide sequence shown in (c₁);
more preferably, the 3' spacer region comprises a sequence shown in any one of (d₁)-(d₂):
(d₁) a nucleotide sequence shown in any one of SEQ ID NOs: 87-89;
(d₂) a sequence having at least 90%, optionally at least 95%, preferably at least 97%, more preferably at least 98%, most preferably at least 99% sequence identity with the nucleotide sequence shown in (d₁).

6. The circular RNA molecule according to claim 5, wherein the circular RNA molecule further comprises a second exon located at the upstream of the 5' spacer region, and a first exon located at the downstream of the 3' spacer region;
preferably, the second exon comprises a sequence shown in any one of (e₁)-(e₂):
(e₁) a nucleotide sequence shown in SEQ ID NO: 91;
(e₂) a sequence having at least 90%, optionally at least 95%, preferably at least 97%, more preferably at least 98%, most preferably at least 99% sequence identity with the nucleotide sequence shown in (e₁);
preferably, the first exon comprises a sequence shown in any one of (f₁)-(f₂):
(f₁) a nucleotide sequence as shown in SEQ ID NO: 90;
(f₂) a sequence having at least 90%, optionally at least 95%, preferably at least 97%, more preferably at least 98%, most preferably at least 99% sequence identity with the nucleotide sequence shown in (f₁).

7. The circular RNA molecule according to any one of claims 1-6, wherein the target polypeptide is a polypeptide having an activity of disease prevention or treatment, preferably a polypeptide having an activity of tumor prevention or treatment; optionally, the polypeptide having the activity of tumor prevention or treatment is one or more selected from: cytokine, an antigen-binding fragment, and an immune checkpoint inhibitor;
optionally, the cytokine is selected from one or more of IL, IFN, TNF, GM-CSF, and M-CSF;
optionally, the antigen-binding fragment specifically binds to one or more tumor antigens as follows: CD19, CD20, CD22, CD30, CD33, CD38, CD123, CD138, CD171, AFP, CEA, PSCA, GD2, NKG2D, BCMA, EGFR, Her2, EGFRvIII, CD171, FAP, IL13Rα2, VEGFR1, VEGFR2, GPC-3, Mesothelin, claudin 18.2, EpCAM, MUC1, MUC16, EPHA2, EPHA3, CD133, and PSMA;
optionally, the immune checkpoint inhibitor is one or more immune checkpoint protein inhibitors as follows: PD-1, PD-L1, PDL2, CTLA4, LAG3, TIM3, TIGIT and CD103.

8. The circular RNA molecule according to any one of claims 1-6, wherein the circular RNA molecule comprises a sequence shown in any one of (g₁)-(g₂):
(g₁) nucleotide sequences shown in SEQ ID NO: 73, and SEQ ID NOs: 76-77;
(g₂) a sequence having at least 90%, optionally at least 95%, preferably at least 97%, more preferably at least 98%, most preferably at least 99% sequence identity with the nucleotide sequences shown in (g₁).

9. A cyclization precursor RNA molecule, wherein the cyclization precursor RNA molecule is cyclized to form the circular RNA molecule according to any one of claims 1-8;
optionally, the cyclization precursor RNA molecule comprises one or more elements as follows:
a 5' homologous arm, a 3' intron, a second exon, a 5' spacer region, a translation initiation element, an expression regulatory element, a coding region, a 3' spacer region, a first exon, a 5' intron and a 3' homologous arm.

10. A recombinant nucleic acid molecule, wherein the recombinant nucleic acid molecule is transcribed to form the cyclization precursor RNA molecule according to claim 9.

11. A recombinant expression vector, wherein the recombinant expression vector comprises the recombinant nucleic acid molecule according to claim 10.

12. A recombinant host cell, wherein the recombinant host cell comprises the circular RNA molecule according to any one of claims 1-8, the cyclization precursor RNA molecule according to claim 9, the recombinant nucleic acid molecule according to claim 10, or the recombinant expression vector according to claim 11.

13. A pharmaceutical preparation or composition, wherein the pharmaceutical preparation or composition comprises the circular RNA molecule according to any one of claims 1-8, the cyclization precursor RNA molecule according to claim 9, the recombinant nucleic acid molecule according to claim 10, the recombinant expression vector according to claim 11, or the recombinant host cell according to claim 12; optionally, the pharmaceutical preparation or composition further comprises one or more pharmaceutically acceptable carriers; preferably, the pharmaceutically acceptable carrier is a liposome material which encapsulates the circular RNA molecule, the cyclization precursor RNA molecule, the recombinant nucleic acid molecule, the recombinant expression vector, or the recombinant host cell;
preferably, wherein the pharmaceutical preparation is a tablet, capsule, injection, spray, granule, powder, suppository, pill, cream, paste, gel, powder, oral solution, inhalant, suspension or dry suspension;
more preferably, the pharmaceutical preparation is the injection;
more preferably, the pharmaceutical preparation is a pharmaceutical preparation which is intratumorally delivered to tumors.

14. The circular RNA molecule according to any one of claims 1-8, the cyclization precursor RNA molecule according to claim 9, the recombinant nucleic acid molecule according to claim 10, the recombinant expression vector according to claim 11, the recombinant host cell according to claim 12, or the composition or pharmaceutical preparation according to claim 13 for use in preventing or treating a disease in a subject, wherein the disease is prevented or treated with the circular RNA molecule, the cyclization precursor RNA molecule , the recombinant nucleic acid molecule, the recombinant expression vector, the recombinant host cell, the pharmaceutical preparation or composition via administration;
optionally, the administration is selected from oral administration, intraperitoneal administration, intravenous administration, intra-arterial administration, intramuscular administration, intradermal administration, subcutaneous administration, transdermal administration, nasal administration, transrectal administration, intratumoral injection, intraluminal retention of tumor, nerve intrathecal injection, intrathecal injection or systemic administration; preferably intratumoral injection;
preferably, the disease is tumor.
